(19) 
Europäisches Patentamt
European Patent Office
Office européen des brevets

(11) **EP 1 881 061 A1**

(12) **EUROPEAN PATENT APPLICATION**
published in accordance with Art. 153(4) EPC

(43) Date of publication:
**23.01.2008 Bulletin 2008/04**

(21) Application number: **06732439.2**

(22) Date of filing: **27.04.2006**

(51) Int Cl.:
*C12M 1/34* (2006.01)  *C12Q 1/04* (2006.01)
*G01N 21/27* (2006.01)  *G01N 21/64* (2006.01)
*G01N 33/48* (2006.01)  *G01N 33/483* (2006.01)

(86) International application number:
**PCT/JP2006/308888**

(87) International publication number:
**WO 2006/120924 (16.11.2006 Gazette 2006/46)**

(84) Designated Contracting States:
**DE FR GB**

(30) Priority: **10.05.2005 JP 2005137854**

(71) Applicant: **Olympus Corporation**
**Tokyo 151-0072 (JP)**

(72) Inventor: **ARAI, Satoshi**
**c/o Olympus Corporation**
**Shibuya-ku**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **CELL OBSERVATION APPARATUS, METHOD OF CELL OBSERVATION AND CELL OBSERVATION PROGRAM**

(57) Viability of cells during culture is accurately determined with minimum damages to cells during long-term culture, and without requiring the introduction of a special dye or gene. When a cell dies during culture without undergoing physical damages, it has a generally circular shape, and ceases its activity while maintaining the generally circular shape. On this account, the circularity of a cellular region $R_{t,m}$ photographed at a plurality of time points is measured as a cell parameter characteristic to the cellular region, and the circularity is compared with the threshold (steps S732 to S735) thereby determining cell viability (steps S736 and S737). This allows accurate determination of cell viability during culture with minimum damages to the cells during long-term culture, and without requiring the introduction of a special dye or gene.

FIG.16

**Description**

TECHNICAL FIELD

The present invention relates to a cell observation apparatus, a cell observation method, and a cell observation program for longitudinally observing living cells during long-term culture.

BACKGROUND ART

Generally, in the fields of new drug development and cell culture, ascertainment of cell death is an important factor for determining the propriety of the components, culture conditions and the like. Cell death is classified into apoptosis (programmed cell death) and necrosis. Apoptosis, which is induced by activation of protease (proteolytic enzyme), is a normal cellular activity involved with, for example, cell development and regulation of cell proliferation. Necrosis is accidental cell death caused by external factors, and refers to cell death caused by a physical damage or toxicity such as light, temperature, or chemical substances.

Conventionally, various methods for detecting cell death have been proposed. Patent Document 1 discloses a concept of longitudinal and optical observation of occurrence of cell death using a cell death test solution. Patent Document 2 discloses a procedure composed of labeling bacteria using two kinds of fluorescence dyes, or one fluorescence dye for labeling living bacteria and another fluorescence dye for labeling dead bacteria, irradiating the bacteria with a pulse excitation light and receiving the emitted fluorescence, and electrically treating the fluorescence thereby precisely counting the number of living or dead bacteria. Patent Document 3 discloses a method of observing dead cells which have been selectively stained, wherein cells are cultured while being bonded to a cell adhesive film pattern. The method facilitates cell counting, and allows accurate observation of the cell survival rate. Accurate ascertainment of the survival rate is important for quantitatively evaluating toxicity in toxicity test.

Patent Document 4 discloses a method of detecting cell death, wherein a gene expressing a special marker protein, which exudes out of cells upon cell death, is introduced into cells, and the behavior of the marker protein is observed. Detection of the marker protein outside the cells represents cell death.

Patent Document 1: Japanese Patent Application Laid-Open No. H5-184579

Patent Document 2: Japanese Patent Application Laid-Open No. 2000-316596

Patent Document 3: Japanese Patent No. 3077628

Patent Document 4: International Publication No. WO 02/052032 Pamphlet

DISCLOSURE OF INVENTION

PROBLEMS TO BE SOLVED BY THE INVENTION

In cases where living cells are longitudinally observed during long-term culture, damages to the cells must be minimized. External environment variability including administration of chemical substances is more or less toxic to cells, and may contribute to shortening of the survival time of the cells. More specifically, reagents for observing cell death may cause cell death. However, the methods described in Patent Documents 1 to 3 require the administration of a dye or reagent to label dead cells, which results in damages to living cells during long-term culture. Accordingly, these methods are not suitable for accurately observing the death process of living cells during the culture process.

The method described in Patent Document 4 employs a system in which cells are labeled with an expressed marker protein. The system is less toxic than a system using a dye or the like, but requires the introduction of a special gene for observation of cell death.

The present invention has been accomplished in view of the above-described problems, and is intended to provide a cell observation apparatus, a cell observation method, and a cell observation program for accurate determination of cell viability during culture with minimum damages to the cells during long-term culture, and without requiring the introduction of a special dye or gene.

MEANS FOR SOLVING PROBLEM

To solve the problems and to achieve the object, a cell observation apparatus as set forth in claim 1 includes a cell recognition unit that recognizes a cellular region representing a cell from cell image data acquired by imaging the cell at a plurality of time points; a cell parameter measurement unit that measures cell parameters characteristic of the cellular region recognized by the cell recognition unit; and a cell viability determination unit that compares the cell parameters measured by the cell parameter measurement unit with thresholds thereby determining cell viability.

The cell observation apparatus as set forth in claim 2 further includes a cell tracking unit that interrelates the cellular regions recognized from the cell image data acquired at different time points, wherein the cell viability determination unit determines the cell viability by repeatedly comparing the cell parameters with the thresholds on the cellular regions interrelated by the cell tracking unit.

In the cell observation apparatus as set forth in claim 3, the cell viability determination unit judges the cell represented by the cellular regions interrelated by the cell tracking unit as not being dead when the same conclusion is derived from the results of the repeated comparisons at a rate below a given rate.

In the cell observation apparatus as set forth in claim 4, the cell viability determination unit judges the cell represented by the cellular regions as not being dead when two or more of the cellular regions are correlated with one of the cellular regions by the cell tracking unit.

In the cell observation apparatus as set forth in claim 5, the cell viability determination unit determines viability of the cell by repeatedly comparing the cell parameters with thresholds, the cell parameters being measured in the cellular regions at a plurality of time points in the imaging period established so as to exceed the mitotic phase in the average cell cycle of the cell under observation.

In the cell observation apparatus as set forth in claim 6, the cell parameters include at least a circularity of the cellular regions.

In the cell observation apparatus as set forth in claim 7, the cell parameters include at least an average intensity of the cellular regions.

In the cell observation apparatus as set forth in claim 8, the cell parameters include at least an edge strength of the cellular regions.

The cell observation apparatus as set forth in claim 9 further includes a culture unit that cultures a cell.

In the cell observation apparatus as set forth in claim 10, the cell is loaded with a fluorescent protein.

In the cell observation apparatus as set forth in claim 11, the cell is loaded with a fluorescent protein which is expressed with no localization.

The cell observation apparatus as set forth in claim 12 further includes an imaging unit that acquires images of the cell at a plurality of time points.

A cell observation program as set forth in claim 13 is for observing a cell in a cell observation apparatus, and causes cell observation apparatus to perform: a cell recognition step of recognizing a cellular region representing a cell from cell image data acquired by imaging the cell at a plurality of time points; a cell parameter measurement step of measuring cell parameters characteristic of the cellular region recognized by the cell recognition step; and a cell viability determination step of comparing the cell parameters measured by the cell parameter measurement step with thresholds thereby determining cell viability.

The cell observation program as set forth in claim 14 further includes a cell tracking step of interrelating the cellular regions recognized from the cell image data acquired at different time points, wherein the cell viability determination step determines the cell viability by repeatedly comparing the cell parameters with the thresholds on the cellular regions interrelated by the cell tracking step.

In the cell observation program as set forth in claim 15, the cell viability determination step judges the cell represented by the cellular regions interrelated by the cell tracking step as not being dead when the same conclusion is derived from the results of the repeated comparisons at a rate below a given rate.

In the cell observation program as set forth in claim 16, the cell viability determination step judges the cell represented by the cellular regions as not being dead when two or more of the cellular regions are correlated with one of the cellular regions by the cell tracking step.

In the cell observation program as set forth in claim 17, the cell viability determination step determines viability of the cell by repeatedly comparing the cell parameters with thresholds, the cell parameters being measured in the cellular regions at a plurality of time points in the imaging period established so as to exceed the mitotic phase in the average cell cycle of the cell under observation.

In the cell observation program as set forth in claim 18, the cell parameters include at least a circularity of the cellular regions.

In the cell observation program as set forth in claim 19, the cell parameters include at least an average intensity of the cellular regions.

In the cell observation program as set forth in claim 20, the cell parameters include at least an edge strength of the cellular regions.

A cell observation method as set forth in claim 21 is for observing a cell in a cell observation apparatus that includes a culture unit which cultures the cell and an imaging unit which images the cell contained in the culture unit. The method includes a cell recognition step of recognizing a cellular region representing the cell from cell image data acquired by imaging, by the imaging unit, the cell cultured in the culture unit at a plurality of time points; a cell parameter measurement step of measuring cell parameters characteristic of the cellular region recognized by the cell recognition unit; and a cell viability determination step of comparing the cell parameters measured by the cell parameter measurement unit with thresholds thereby determining cell viability.

The cell observation method as set forth in claim 22 further includes a cell tracking step of interrelating the cellular regions recognized from the cell image data acquired at different time points, wherein the cell viability determination step determines the cell viability by repeatedly comparing the cell parameters with the thresholds on the cellular regions interrelated by the cell tracking step.

EFFECT OF THE INVENTION

The cell observation apparatus, cell observation method, and cell observation program according to the present invention determine cell viability by comparing cell parameters characteristic of cellular regions, which have been photographed and measured at a plurality of time points, with thresholds. Accordingly, they are capable of accurately determining cell viability during culture with minimum damages to the cells during long-term culture and without requiring the introduction of a special dye or gene. In addition, the cell parameters of the cellular regions interrelated with each other by cell tracking are repeatedly compared with thresholds thereby determining cell viability, so that viability of the cells is more accurately determined.

BRIEF DESCRIPTION OF DRAWINGS

FIG. 1 is a schematic block diagram showing a structure example of a cell observation apparatus according to an embodiment of the present invention;
FIG. 2 is a horizontal sectional view showing a structure example of the culture unit;
FIG. 3 is a longitudinal elevation view showing a structure example of a culture unit;
FIG. 4 is a perspective view showing a structure example of a current plate;
FIG. 5 is a cross sectional view showing an insulation structure example of the boundary between a culture unit and an imaging unit;
FIG. 6 is an explanatory drawing showing an image example of cells during culture acquired by fluorescence imaging;
FIG. 7 is an explanatory drawing showing the process of imaging of a plurality of visual fields (visual fields 1 to N);
FIG. 8 is an explanatory drawing showing an example of timing of imaging of the visual fields 1 to N;
FIG. 9 is a schematic flowchart showing an example of image data processing;
FIG. 10 is a drawing showing weighting by a sharpened filter;
FIG. 11 is a schematic flowchart showing an example of the first technique of regional integration;
FIG. 12 is a schematic flowchart showing an example of the second technique of regional integration;
FIG. 13 is an explanatory drawing showing an example of measured cell parameters recorded in the recording unit;
FIG. 14 is an explanatory drawing showing the calculated scores on possible combination of m and n;
FIG. 15 is a schematic flowchart showing the first procedure of cell viability determination;
FIG. 16 is a schematic flowchart showing the second procedure of cell viability determination;
FIG. 17 is a schematic flowchart showing the third procedure of the cell viability determination;
FIG. 18 is a schematic flowchart showing the fourth procedure of the cell viability determination;
FIG. 19 is a schematic flowchart showing the fifth procedure of the cell viability determination;
FIG. 20 is an explanatory drawing showing an indication example of a processing result; and
FIG. 21 is an explanatory drawing showing an example of highlighting.

EXPLANATIONS OF LETTERS OR NUMERALS

101    Culture unit
201    Imaging unit
306    Cell recognition unit
307    Parameter measurement unit
308    Cell tracking unit
313    Cell viability determination unit

BEST MODE(S) FOR CARRYING OUT THE INVENTION

A suitable embodiment according to the present invention is described below in detail with reference to the following drawings. The cell observation apparatus according to an embodiment of the present invention images a plurality of living cells loaded with a fluorescent protein during long-term culture, recognizes respective cellular regions, individually measures cell parameters characteristic of respective cells while tracking the change of position over time, and then determines cell viability.

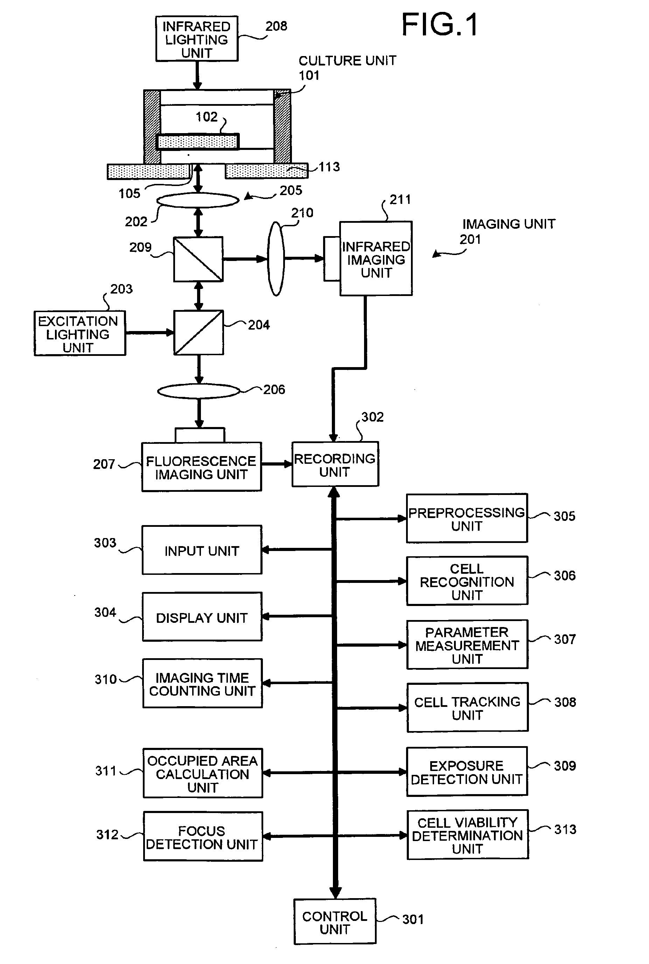

FIG. 1 is a schematic block diagram of a cell observation apparatus according to an embodiment of the present invention. The cell observation apparatus according to the present embodiment is schematically composed of a culture unit 101 for culturing cells, an imaging unit 201 for imaging cells contained in the culture unit 101, a control unit 301 for controlling the processing and operation of the whole of the cell observation apparatus, a recording unit 302 for temporality or permanently recording all of data, which include image data acquired in the imaging unit 201 and processed data, an input unit 303 receiving input of various information, and a display unit 304 for displaying and showing the operator various information such as image information, a preprocessing unit 305, a cell recognition unit 306, a parameter measurement unit 307, a cell tracking unit 308, an exposure detection unit 309, an imaging time counting unit 310, an occupied area calculation unit 311, a focus detection unit 312, and a cell viability determination unit 313. The units 302 to 313 are connected to the control unit 301, and controlled by the control unit 301. FIG. 1 does not specifically show the control over the culture unit 101 and imaging unit 201 by the control unit 301 and connection between them. Each processing by the control unit 301, preprocessing unit 305, cell recognition unit 306, parameter measurement unit 307, cell tracking unit 308, exposure detection unit 309, imaging time counting unit 310, occupied area calculation unit 311, focus detection unit 312, and cell viability determination unit 313 is carried out with necessary data written as appropriate by the CPU installed in the cell observation apparatus into a storage device such as a RAM on the basis of processing programs stored in a memory such as a ROM.

In the first place, the culture unit 101 is further described below. A slide glass 102 retaining a plurality of living cells C loaded with a fluorescent protein, which is expressed with no localization, is installed in the culture unit 101. The culture unit 101 has the same structure as, for example, the culture vessel disclosed in Japanese Patent Application Laid-Open No. 2004-113175. In this case, the fluorescent protein is not specifically limited as long as it does not cause localization, and may be a general fluorescent protein derived of jellyfish origin such as pEGFP-N1 manufactured by BD Bioscience Clontech.

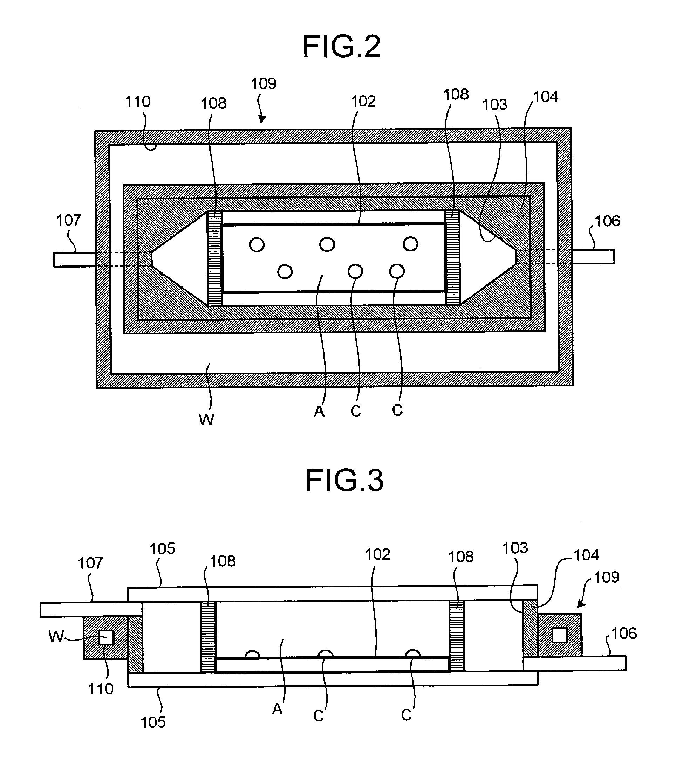

FIG. 2 is a horizontal sectional view showing a structure example of the culture unit 101, and FIG. 3 is a longitudinal elevation view showing a structure example of the culture unit 101. The culture unit 101 as a culturing means is, as shown in FIG. 2 and FIG. 3, composed of a cabinet 104 which is made of a highly heat-conductive material such as stainless steel or aluminum and has a vertical through hole 103 having enough space for the slide glass 102, observation windows 105 composed of two glass plates which are optically smooth and block the vertical through hole 103 of the cabinet 104, a culture medium feeding pipe 106 for feeding a culture medium A into the cabinet 104, a culture medium discharging pipe 107 for discharging the culture medium A no longer required from the cabinet 104, and two current plates 108 provided in the cabinet 104 at the inlet and outlet for the culture medium A.

In order to healthily grow the living cells C, it is desirable that the fresh culture medium A be fed constantly all over the slide glass 102. However, if the current of the culture medium A is too fast, the living cells C landed on the slide glass 102 may fall off. Therefore, according to the present embodiment, the current plates 108 are provided in the vicinity of the pipes 106 and 107 thereby allowing uniform division and recover of the culture medium A current.

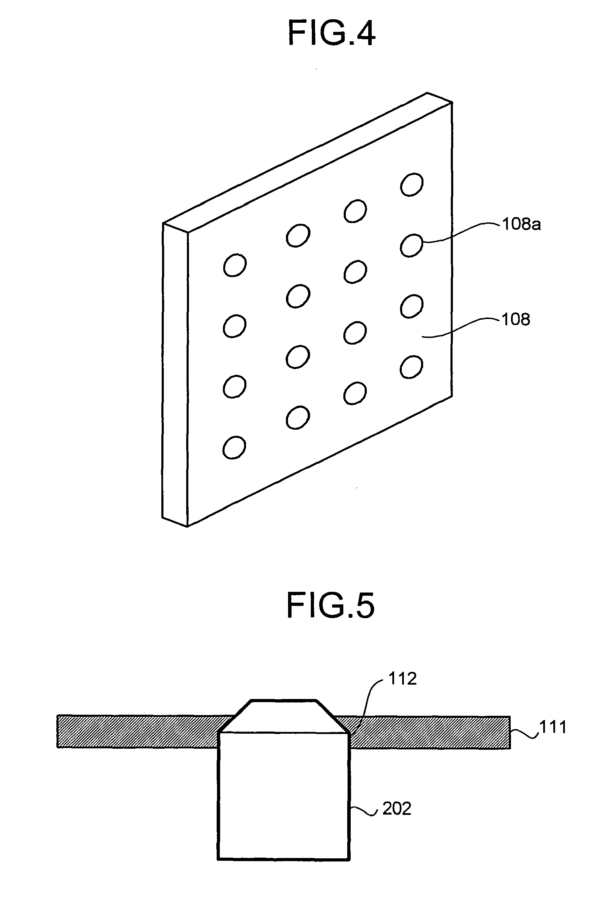

FIG. 4 is a perspective view showing a structure example of the current plate 108. The current plates 108 is, as shown in FIG. 4, a porous member having a plurality of through holes 108a in the thickness direction. The current plate 108 on the inlet side divides the culture medium A flowing from the culture medium feeding pipe 106 into currents passing through a plurality of through holes 108a, and the current plate 108 on the outlet side divides the culture medium A rushing toward the culture medium discharging pipe 107 into currents passing through a plurality of through holes 108a. Thus, the concentrated current is converted into divided currents, so that the culture medium A is flown at a constant flow rate and a constant quantity in the vicinity of the slide glass 102 having thereon the living cells C.

The culture unit 101 is equipped with a temperature control unit 109, and a hot water channel 110 for flowing hot water W is formed around the culture unit 101. By circulating the hot water W in the hot water channel 110, heat of the hot water is transferred to the culture medium A through the cabinet 104. At that time, temperature information from a temperature sensor (not shown) is transferred to the control unit 301 at regular time intervals, and the control unit 301 controls the temperature and flow rate of the hot water W thereby maintaining the temperature in the culture unit 101 at $37 \pm 0.5°C$.

In addition, the pH information of the culture medium A is transferred to the control unit 301 at regular time intervals by a pH sensor (not shown), and the control unit 301 controls the $CO_2$ concentration in the culture medium A thereby maintaining the pH of the culture medium within a given range.

The culture medium before use is stored in a culture medium storage unit (not shown), and cooled to and kept at about 4°C by a cooling mechanism (not shown) thereby suppressing deterioration over time. The cooled culture medium is warmed to about 37°C by a culture medium warming mechanism (not shown), and then fed into the cabinet 104 through the culture medium feeding pipe 106.

The culture medium discharged through the culture medium discharging pipe 107 is stored in a waste water storage unit (not shown). The discharged culture medium may be partially mixed with a fresh culture medium to be fed into the cabinet 104. In this case, the impact on cells incident to the replacement of the culture medium is reduced, which is more suitable to long-term culture.

FIG. 5 is a cross sectional view showing an insulation structure example of the boundary between the culture unit 101 and the imaging unit 201. An insulation unit 111 as an insulation means prevents heat transfer from the culture unit 101 to the imaging unit 201. The insulation unit 111 may be installed in any position to insulate the culture unit 101 from the imaging unit 201, but according to the present embodiment, the insulation unit 111 is installed between the cabinet 104 of the culture unit 101 and an imaging element composing the imaging unit 201. The insulation unit 111 is a sheet made of a highly insulative and elastic member such as rubber, silicon, or polyurethane, and has a through hole 112 having an approximately same diameter as an objective lens 202. The culture unit 101 is optically connected to the objective lens 202 via the through hole 112 thereby freely exchanging light beams. The heat emitted by the culture unit 101 is mostly blocked by the insulation unit 111. Generally, an optical system is adjusted on the assumption that it is used at about 25°C. Therefore, if the system is heated by the heat from the culture unit 101, it cannot exhibit expected performance. In particular, the noise of a solid-state imaging device such as CCD composing the imaging unit 201 increases to deteriorate the SN ratio as the temperature increases. Therefore, in order to capture a weak fluorescence, the temperature must be kept as low as possible with no dew condensation.

As exemplified by the culture unit 101, the culture medium in the culturing means is more preferably replaceable, but a general well plate may be used for cell observation. However, in cases where a general well plate is used, the culture medium cannot be replaced with the environmental conditions maintained, so that the culture period is shortened because of the influence of the deterioration of the culture medium incident to cellular metabolism in comparison with the case using the culture unit 101.

Through the use of the above-described structure, the temperature and pH of the culture medium A in the culture unit 101 are maintained virtually constant. The living cells used as the measuring sample are, for example, HeLa cells. HeLa cells are derived from cervical cancer, and have been widely used in new drug toxicity test or the like. The kind of the fluorescent protein to be introduced may be changed according to the contents of the assay.

In the next place, the imaging unit 201 is further described below. The imaging unit 201 is composed of an excitation lighting unit 203, a dichroic mirror 204, an objective optical system 205, an imaging optical system 206, a fluorescence imaging unit 207, an infrared lighting unit 208, a dichroic mirror 209, an imaging optical system 210, and an infrared imaging unit 211. More specifically, the imaging unit 201 according to the present embodiment has a fluorescence imaging system and an infrared imaging system.

The light emitted from the excitation lighting unit 203 is reflected from the dichroic mirror 204, and radiated over the slide glass 102 through the objective optical system 205 including the objective lens 202, and the observation windows 105. The fluorescent protein contained in the living cells C on the slide glass 102 is excited by the irradiated light to emit a fluorescence, and both of the reflected excitation light and fluorescence are ejected from the observation windows 105. The ejected light passes through the objective optical system 205 again and reach the dichroic mirror 204 which transmits the fluorescence but blocks the reflected excitation light. The fluorescence passes through the dichroic mirror 204, and is enlarged and projected by the imaging optical system 206 to form an image on a solid-state imaging device such as CCD or CMOS composing the fluorescence imaging unit 207 as an optical cell imaging means.

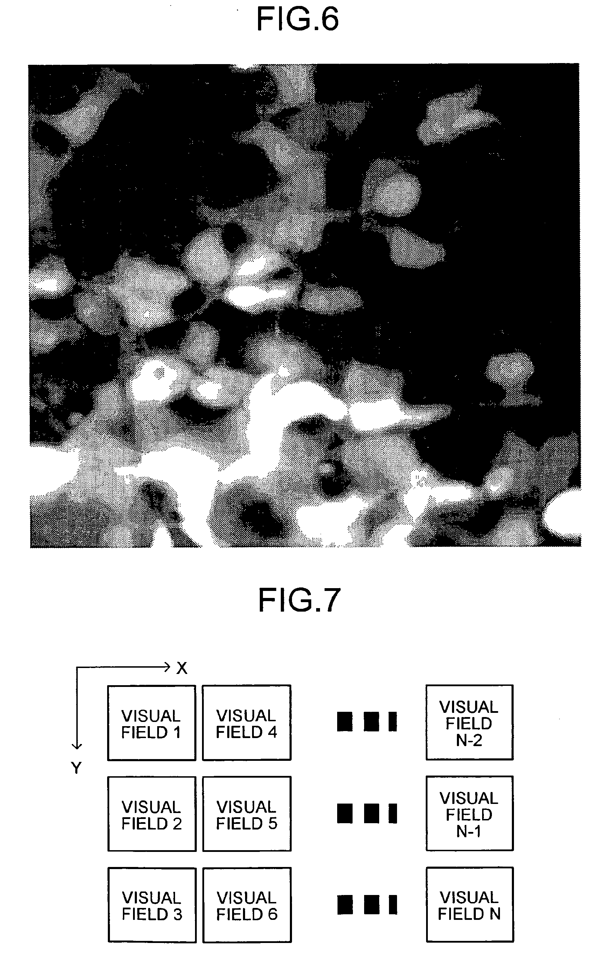

The fluorescence image of the measuring sample is converted into image data by the solid-state imaging device composing the fluorescence imaging unit 207, and temporarily or permanently recorded in the recording unit 302 under control of the control unit 301. FIG. 6 is an explanatory drawing showing an image example of cells during culture acquired by fluorescence imaging.

The light emitted from the infrared lighting unit 208 is radiated over the slide glass 102 through one observation window 105, and the transmitted light is ejected from the other observation window 105. The ejected light passes through the objective optical system 205 and reaches a dichroic mirror 209 which reflects infrared light. The reflected infrared light is enlarged and projected by the imaging optical system 210 to form an image on a solid-state imaging device such as CCD or CMOS composing the infrared imaging unit 211. The infrared image of the measuring sample is converted into image data by a solid-state imaging device such as CCD or CMOS composing the infrared imaging unit 211, and temporarily or permanently recorded in the recording unit 302 under control of the control unit 301.

In general, a fluorescent protein cannot be uniformly introduced into all cells, and even if introduced, the fluorescent protein may not be immediately expressed. Therefore, a means for longitudinal and stable observation of cells in their entirety is necessary, and the means is an infrared image in the present embodiment. Through the use of an infrared image, even if the fluorescent protein in the measuring sample is scarcely or not expressed in the initial state, the object range can be examined and adjusted under observation of the cell image.

Since infrared light is less phototoxic to living cells than visible light, the cell activity is maintained for a longer period of time in infrared light in comparison with the case of imaging in visible light. In addition, visible light over the full range can be used as the excitation light for fluorescence imaging, which eases constraints on usable fluorescent proteins.

Thus, the imaging unit 201 according to the present embodiment images the living cells C on the slide glass 102 using the fluorescence imaging unit 207 and the infrared imaging unit 211 thereby acquiring the image data of the living cells C image. According to the present embodiment, the fluorescence imaging unit 207 automatically carries out imaging at given time intervals under control of the control unit 301. When a user wants to observe the cells as necessary, the user can observe the living cells C at desired times using the infrared imaging unit 211. Imaging may be carried out at desired times by the user using the infrared imaging unit 211, or may be carried out under control of the control unit 301 in synchronism with the imaging by the fluorescence imaging unit 207. This facilitates correlating infrared images with fluorescence images, and also facilitates correlating the living cells C contained in the images with each other. As a result of this, the living cells C are efficiently observed during long-term culture with reduced decrease in cell activity. The display unit 304 may have a function of displaying the time taken for fluorescence imaging and infrared imaging.

The structure according to the present embodiment is composed of the fluorescence imaging unit 207 and the infrared imaging unit 211, so that it can carry out fluorescence imaging and infrared imaging in parallel, and thus remarkably shorten the time required for imaging in comparison with imaging requiring switching of the units, and requires no driving unit for switching.

Phase contrast observation can be carried out in place of transmission observation by adding a ring aperture to the infrared lighting unit 208, and inserting a phase plate into the light channel extending from the dichroic mirror 209 to the imaging optical system 210. The phase contrast observation offers higher contrast images than transmission observation.

Differential interference observation can be carried out in place of transmission observation by inserting a polarizer and a DIC (differential interference contrast) element into the infrared lighting unit 208, and inserting a DIC slider and an analyzer into the light channel extending from the dichroic mirror 209 to the imaging optical system 210. Differential interference observation offers higher contrast images than transmission observation.

Imaging and recording of the visual fields (imaging ranges) are repeated a given number of times with the relative positions of the slide glass 102, fluorescence imaging unit 207, and the solid-state imaging device composing the infrared imaging unit 211 changed by a stage transfer mechanism 113, and thus the cell image data is acquired. In cases where imaging is carried out with a plurality of visual fields switched over one by one, the stage position in each visual field is recorded, and the stage position is reproduced by the stage transfer mechanism 113 before the second and subsequent imaging of the visual fields. FIG. 7 is an explanatory drawing showing the process of imaging of a plurality of visual fields (visual fields 1 to N). The position of each visual field is arbitrary, and not particularly limited to a lattice shape. The visual fields may overlap one another.

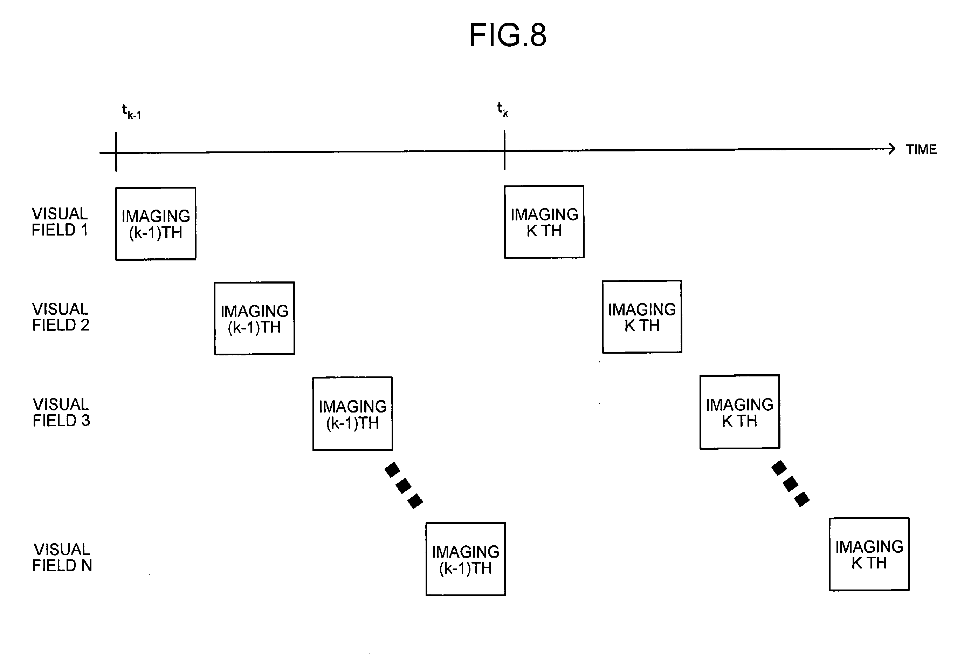

FIG. 8 is an explanatory drawing showing an example of timing of imaging of the visual fields 1 to N. Imaging of the visual fields 1 to N is carried out in a given order at virtually constant intervals.

Whether the exposure during image data acquisition is appropriate or not is detected by an exposure detection unit 309. If the exposure during imaging is inappropriate, imaging of the inappropriately exposed area is carried out again immediately or after imaging of any other observation area is completed. In this case, the exposure conditions may be changed. In the same manner, whether the focusing during image data acquisition is appropriate or not is detected by a focus detection unit 312. If the focusing during imaging is inappropriate, imaging of the inappropriately focused area is carried out again immediately or after imaging of any other observation area is completed. In this case, the focusing conditions may be changed.

The culture medium A circulates in the culture unit 101, and the circulation may be temporarily halted in time with imaging. This prevents fluctuations in the background during imaging caused by the circulation of the culture medium.

The number of times of imaging in a given visual field may be counted by the imaging time counting unit 310 as a means for recognizing the time point of imaging. In this case, after a given number (frame) of imaging of a given visual field, the image is displayed on the display unit 304 as a notification means, and then the operator is required to examine the content of the image. If the content is judged as having no problem, the processing is continued, and if judged having any problem, instructions regarding resetting of the imaging conditions are given by the operator. Alternatively, the processing may be halted. If no response is given from the operator in a given amount of time, whether the processing is continued or halted is determined according to the established instructions.

According to the present embodiment, the number of times of imaging by the fluorescence imaging unit 207 (or the infrared imaging unit 211) is counted, and the operator is required to confirm the image after a given number of times of imaging. Alternatively, the image may be examined not only in terms of the number of times of imaging, but also by a means for measuring the lapse of a given period from the initiation of observation as a means for recognizing the time point of imaging, in which, for example, information regarding the time of cell image data acquisition is acquired, and when the time exceeds a predetermined period, acquisition of cell image data at a given time point is recognized.

If the culture period is prolonged, the cells during culture may proliferate or die beyond expectations, or the image intensity may excessively increase, which can result in the failure to acquire appropriate images for cell observation at a certain time point. For these reasons, as described above, the operator is notified about the lapse of a given time, or the processing is halted thereby allowing the operator to examine the acquired image or the image at the time point for appropriately carrying out the subsequent cell observation.

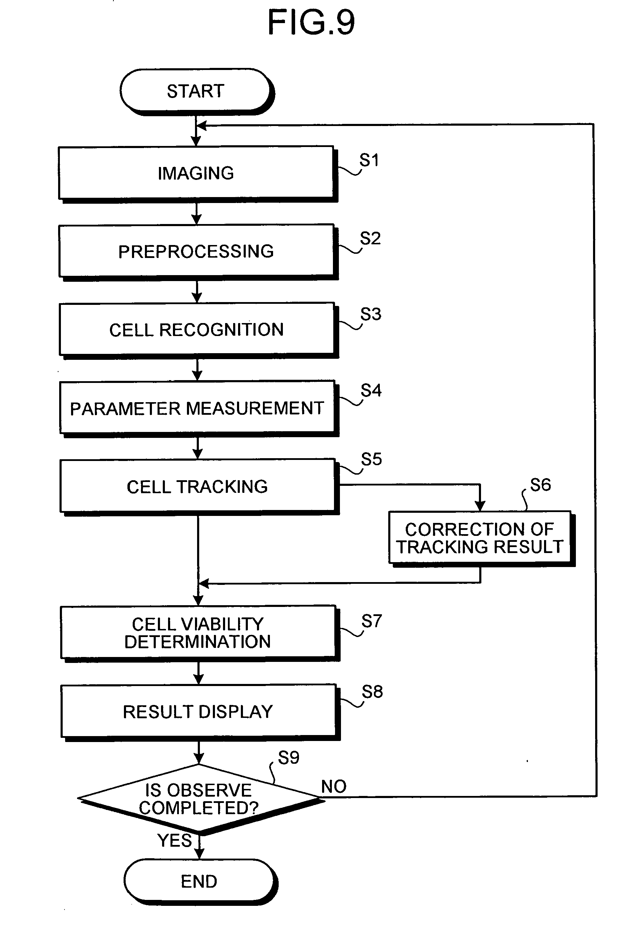

In the next place, among the acquired images, the image data acquired by the fluorescence imaging unit 207 is processed as described below. FIG. 9 is a schematic flowchart showing an example of image data processing carried out by the preprocessing unit 305 and others under control of the control unit 301. Following the above-described cell imaging (step S1) by the imaging unit 201, preprocessing is carried out by the preprocessing unit 305 (step S2), and the cells are recognized by the cell recognition unit 306 as a means for recognizing cells (step S3). Subsequently, the cell parameters characteristic of the recognized cells are measured by the parameter measurement unit 307 as a means for measuring cell parameters on the basis of the cell image data (step S4). Further, the cell tracking unit 308 as a cell tracking means identifies the cells recognized from the cell image data acquired at different time points on the basis of the cell parameters (step S5). Alternatively, the tracking result is further corrected (step S6). Subsequently, the cell viability determination unit 313 as a means for determining cell viability determines cell viability (step S7). Thereafter, the display unit 304 displays the obtained results including the tracking result, and viability determination result (step S8). The above processing steps are repeated in the same manner until the observation is completed (step S9: Yes).

The processing in step S1 (or steps S1 and S2) may be previously carried out at a plurality of time points, wherein the processing in step S2 and subsequent steps (or step S3 and subsequent steps) for the image data acquired at the time points is carried out all at once later. Like this, when image data are previously acquired at a plurality of time points and processed all at once later, the device structure is more simple, and higher responsiveness and stability are provided with a low-cost calculator, in comparison with imaging carried out in parallel with image data processing.

The processing steps are detailed one by one. In step S2, the acquired cell image data recorded in the recording unit 302 is processed in the preprocessing unit 305 as follows. In the first place, a low-pass filter of edge preservation type is applied to the cell image data. The low-pass filter of edge preservation type smoothens the areas other than edges while suppressing deterioration of the high frequency component of spacial frequency in the edge areas, and is suitable to the present technique because it removes noises while keeping the contour information of the cells.

As a filter satisfying the requirement, a bilateral filter (see Tomasi & Manduchi, "Bilateral Filtering for Gray and Color Images", Proceedings of the 1998 IEEE International Conference on Computer Vision, Bombay, India) is known, which is used in the present technique.

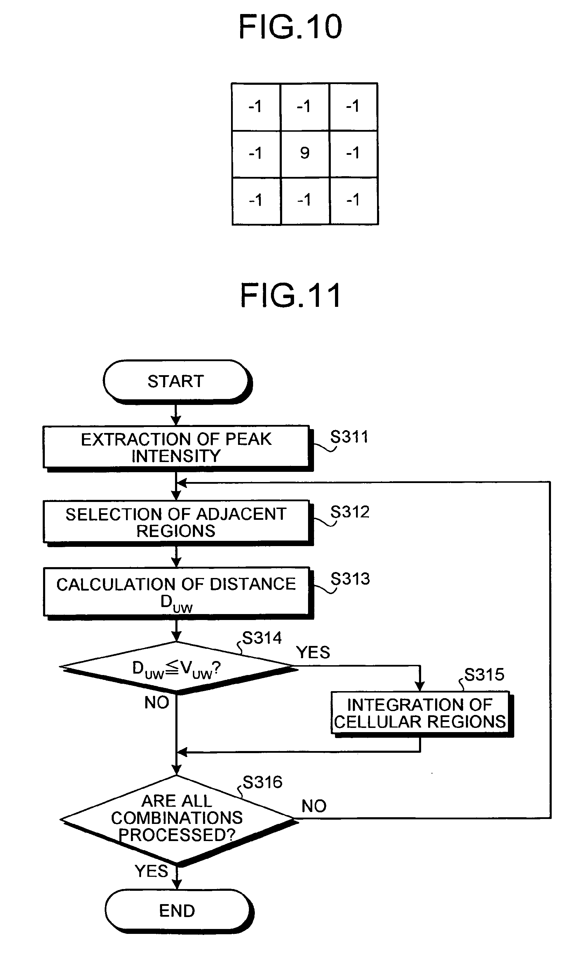

Subsequently, a sharpening filter for highlighting edges is applied to the cell image data after being subjected to the low-pass filter of edge preservation type. The sharpening filter is a filter for obtaining the total by weighting the target pixel and neighboring 8 pixels, for example as shown in FIG. 10. The application is repeatedly carried out for each pixel thereby achieving sharpening.

In step S3, the preprocessed cell image data is analyzed in the cell recognition unit 306 by the following procedure, wherein the regions occupied by the cells are recognized. According to the procedure, the regions occupied by the cells can be recognized not only when the cells are scattered with no contact among them, but also when the cells are compacted in contact with each other. The procedure is also applicable to the case where the edges of the cellular regions are obscure.

In the first place, the image is divided into regions containing concentrated high-intensity pixels. In a general fluorescence image, a cell seems like a lump of high-intensity pixels, so that the division into regions (lumps) containing concentrated high-intensity pixels is equivalent to the division of the image into respective cellular regions.

Watershed regional division is known as a processing procedure satisfying the above requirement. In the present embodiment, the watershed regional division method is used as the procedure for cell recognition (see Vincent & Soille, "Watersheds in Digital Spaces: An Efficient Algorithm Based on Immersion Simulations", IEEE TRANSACTIONS ON PATTERN ANALYSIS AND MACHINE INTELLIGENCE, VOL. 13, NO. 6, June 1991). According to the watershed regional division method described in the original paper, an image is divided into regions containing concentrated low-intensity pixels. In the present embodiment, the intensity is reversed, so that the image is divided into high-intensity regions. The divided regions correspond to respective cellular regions.

According to the properties of the adjacent cellular regions, a plurality of cellular regions may be integrated into one new cellular region. The watershed regional division tends to divide the image into small regions, so that the integration improves the quality of the recognition result.

The first technique of regional integration is described with reference to FIG. 11. FIG. 11 is a schematic flowchart showing an example of the first technique of regional integration. In the first place, the maximum intensity in respective cellular regions, or the peak intensity is determined (step 5311). In the next place, adjacent two cellular regions are selected arbitrarily (step S312), and the distance $D_{uw}$ between the peaks along the line segment connecting the peaks is determined (step S313). The distance $D_{UW}$ is calculated by Formula (1):

[Formula 1]

$$D_{uw} = \sum_{P} \left( I(P) - \overline{I(P_s)} \right) \tag{1}$$

I(P) represents the intensity of the pixel P in the image after application of the low-pass filter of edge preservation type, /I(P_S) represents the average intensity of two peaks in the image after application of the low-pass filter of edge preservation type, and $\Sigma$ represents the integration of all the pixels on the line segment connecting the peaks.

In step S313, the distance $D_{UW}$ between the peaks is determined for all the combinations of adjacent cellular regions, and then in step S314, the distance $D_{UW}$ is compared with a given threshold $V_{UW}$. When the comparison result indicates that $D_{UW}$ is equal to or lower than the given threshold $V_{UW}$ (step S314: Yes), the cellular regions are integrated into one region (step S315). The processing is repeated in the same manner until all the combinations are processed (step S316: Yes).

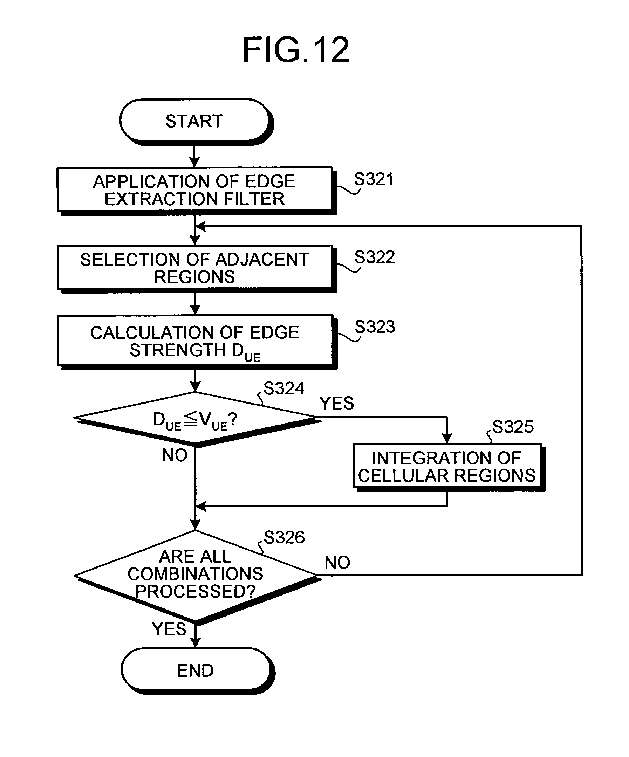

The second technique of regional integration is described with reference to FIG. 12. FIG. 12 is a schematic flowchart showing an example of the second technique of regional integration. In the first place, an edge extraction filter such as a Sobel filter is applied to the output result of the low-pass filter of edge preservation type thereby acquiring an edge image (step S321). Adjacent two arbitrary cellular regions are selected in consideration of the borderline between the arbitrary adjacent cellular regions (step S322), and the edge strength $D_{UE}$ is determined by Formula (2) (step S323):

[Formula 2]

$$D_{UE} = \sum_{P} E(P) \tag{2}$$

Here, E(P) is the intensity of the pixel P in the edge image, and $\Sigma$ represents the integration of all the pixels contained in the borderline between the cellular regions.

In step S323, the edge strength $D_{UE}$ is determined for all the combinations of adjacent cellular regions, and in the subsequent step S324, the edge strength $D_{UE}$ is compared with a given threshold $V_{UE}$. If the comparison result indicates that $D_{UE}$ is equal to or lower than the given threshold $V_{UE}$ (step S324: Yes), the cellular regions are integrated into one region (step S325). The processing is repeated in the same manner until all the combinations are processed (step S326: Yes).

The first and second techniques of regional integration may be independently used, or successively used in an arbitrary order. In addition, the cellular regions may be verified using intensity information. In that case, the pixel exhibiting the maximum intensity is determined in each of the divided cellular regions, and if the intensity is lower than the given threshold $V_{tmin}$, the region is judged as not a cellular region, and excluded together with accompanying pixels from the objects of subsequent processing. As a result of this, cells containing an insufficient amount of or insufficiently expressed fluorescent protein, and background regions other than cells are removed.

In addition, the intensity of each pixel in a cellular region may be compared with a given threshold $V_{pmin}$ thereby excluding pixels having a lower intensity than the threshold $V_{pmin}$ from the cellular region. The excluded pixels are not used for the subsequent processing. As a result of this, low intensity areas having a low SN ratio are excluded from the cellular region, which allows more accurate recognition of the boundary shape of the cellular region. The resultant cellular region and the set of pixels contained in the region are recorded in the recording unit 302.

The infrared image acquired by the infrared imaging unit 211 may be used for recognition of cellular regions. In cases where the infrared image is a phase contrast image, the intensity of the region containing a cell is observed as being different from that of the background. Accordingly, cellular regions are recognized by comparing the intensity of the pixels in the image with the typical intensity $P_{BG}$ of the background, extracting the pixels having a difference larger than the given threshold $V_{PG}$, and then integrating adjacent pixels by general labeling.

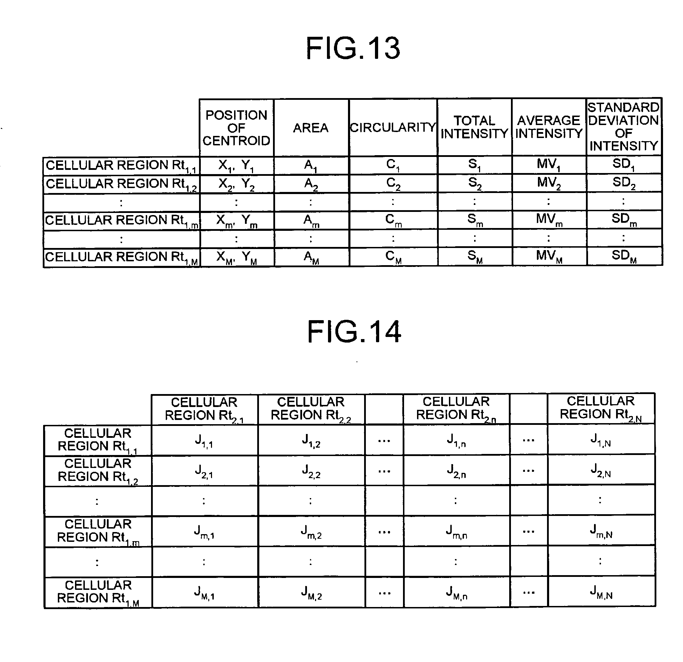

Returning to the processing shown in FIG. 9, in step S4, the cellular regions recognized by the cell recognition unit 306 are individually measured for the cell parameters by the parameter measurement unit 307, and the measurement results are recorded in the recording unit 302. FIG. 13 is an explanatory drawing showing an example of measured cell parameters recorded in the recording unit 302. M represents the number of recognized cellular regions. According to the present embodiment, cell parameters include, for example, the position of centroid, area, circularity, total intensity, average intensity, and standard deviation of intensity as the measurement items, and they are recorded in the recording unit 302 in association with the cell image data and cellular regions. According to the contents of the assay, general measurement items such as perimeter, Feret's diameter, length, width, and maximum intensity may be added.

According to the present embodiment, the total area of all the cells in the image, more specifically the area equivalent to the cell occupancy representing the degree of occupation of the image by the cellular regions is calculated by the occupied area calculation unit 311 as a calculation means, and when the area in the image occupied by the cellular regions exceeds a given proportion with reference to the image area, the event is notified to the control unit 301. In this case, the control unit 301 may notify the operator about the event according to the predesignated setting through the display unit 304 as a notification means, and may alter the control conditions for the culture unit 101. Alternatively, the notification may be ignored. The function is effective for notifying the lack of spaces on the medium during cell culture, because prolonged culture may cause the decrease in spaces on the medium because of cell proliferation or the like.

The occupied area calculation unit 311 determines the image area occupied by the cellular regions in terms of cell occupancy. The cell image may be a fluorescence image or infrared image. In cases where the target is a fluorescence image, the areas of the cellular regions have been measured by the parameter measurement unit 307, so that the image area occupied by the cellular regions is determined by summating the area of all the cellular regions in the image. In cases where the target is an infrared image, the intensity of the region containing cells is observed as being different from that of the background. Accordingly, the image area occupied by the cellular regions can be determined by comparing the intensity of the pixels in the image with the typical intensity $P_{BG}$ of the background, extracting the pixels having a difference larger than the given threshold $V_{PG}$, and then calculating the number of pixels extracted from the image.

By the above procedure, a single cell image including a plurality of cell images is measured for the respective cellular regions, and then measured for the cell parameters of each region. The cell parameters are accumulated with the lapse of time by repeating the acquisition of cell images and parameter measurement at given time intervals $\Delta t$.

With the processing so far, the cell parameters measured at different times have not been correlated with each other, and cannot be regarded as longitudinally measured. Therefore, the cellular regions in cell images acquired at different times must be correlated with each other thereby associating the cell parameters with each other.

The cellular regions are correlated with each other in the cell tracking unit 308 as follows, as the processing in steps S5 and S6. The cellular region recognized at the time t1 is expressed as Rt1,m, and the cellular region recognized at the time t2 is expressed as Rt2,n. The time t2 is later than the time t1 in time sequence. m and n are identification numbers of the cellular regions with no overlap in the same image, satisfy $1 \leq m \leq M$ and $1 \leq n \leq N$, respectively, wherein M and N represent the number of cellular regions recognized at the times t1 and t2, respectively.

The evaluation function regarding the relationship between the two cellular regions Rt1,m and Rt2,n is defined by Formula (3). The smaller the evaluation value J1 calculated by Formula (3), the closer the relationship between the two regions, and the higher the possibility of denoting the same cell.

[Formula 3]

$$J_1 = J_1(R_{t1,m}, R_{t2,n}) = k_d \delta_d + k_a \delta_a + k_c \delta_c \qquad (3)$$

$\delta_d$: Distance between centroids

$\delta_a$: Difference in area

$\delta_c$: Difference in circularity

$k_d, k_a, k_c$: Given weighting coefficients

FIG. 14 is an explanatory drawing showing the calculated scores on possible combinations of m and n. In FIG. 14, $J_1(R_{t1,m}, R_{t2,n})$ is abbreviated as $J_{m,n}$, for convenience of description.

The region $R_{t2,n^{\wedge}}$ at the time $t_2$ corresponding to the region $R_{t1,m}$ at the time $t_1$ is determined according to Formula (4). More specifically, $R_{t2,n^{\wedge}}$ is the region at the time $t_2$ which minimizes the evaluation value $J_1$ in relation to the region $R_{t1,m}$.

[Formula 4]

$$R_{t_2,\hat{n}} : \text{where } J_1\left(R_{t_1,m}, R_{t_2,\hat{n}}\right) = \min_{1 \le n \le N} J_1\left(R_{t_1,m}, R_{t_2,n}\right) \quad\quad (4)$$

If there are a plurality of n^ values which minimize the evaluation value $J_1$, the second evaluation function expressed by Formula (5) is applied to the values thereby determining the combination which minimizes the evaluation value $J_2$. If there are a plurality of combinations which minimize the second evaluation value $J_2$, a message to the operator is displayed on the display unit 304, and the operator selects an optimal combination and inputs it from the input unit 303, and then the correlation is established on the basis of the input result.
[Formula 5]

$$J_2 = J_2(R_{t1,m}, R_{t2,n}) = k_s\delta_s + k_m\delta_m + k_v\delta_v \quad\quad (5)$$

$\delta_s$: Difference in total intensity
$\delta_m$: Difference in average intensity
$\delta_v$: Difference in standard deviation of intensity
$k_s$, $k_m$, $k_v$: Given weighting coefficients

Either of the evaluation values $J_1$, $J_2$, not both, may be determined, thereby accelerating the processing. Alternatively, all of the correspondences which minimize the evaluation value $J_1$ or $J_2$ may be recorded without the display of the message to the operator and the input step by the operator.
The region $R_{t1,m}$ at the time $t_1$ and the region $R_{t2,n^}$ at the time $t_2$ can be regarded as the result of recognition of the same cell at different times, so that the measured cell parameters of them can be regarded as measured values on the same cell obtained at different times. Accordingly, the cell parameters are recorded in the recording unit 302 as a recording means in association with the cell image, cellular regions, correlation between cellular regions, and time information, and thus the longitudinal parameter measurement is completed.
With the lapse of time, if another fluorescent protein is expressed to emit a fluorescence, if cells out of the observation screen move into the observation screen, if a plurality of overlapped cell are separated, or if cells are divided, the number of the cellular regions recognized in the cell recognition unit 306 increases, so that the cellular region at the time $t_1$ corresponding to the cellular region at the time $t_2$ may not exist, or a plurality of cells at the time $t_2$ may correspond to one cell at the time $t_1$.
In addition, with the lapse of time, if the fluorescence intensity of the fluorescent protein decreases, if cells on the observation screen move out of the observation screen, if a plurality of cells are overlapped, or if cells die out, the number of the cellular regions recognized in the cell recognition unit 306 decreases, so that the cellular region at the time $t_2$ corresponding to the cellular region at the time $t_1$ may not exist, or a plurality of cells at the time $t_2$ may correspond to one cell at the time $t_1$.
If there is no corresponding cellular region, a flag indicating the absence of corresponding region is recorded. If a plurality of cellular regions correspond to one cellular region, all the correspondences are recorded. A message to the operator may be displayed on the display unit 304 thereby correcting the correspondence on the basis of the input from the operator. Data representation for recording the correspondence between a plurality of cellular regions employs a tree structure in which the height and nodes correspond to the time and cellular regions, respectively. A graph structure having higher flexibility in representation may be used.
The association between the cellular region may be modified as follows. According to a first modification, if the minimum evaluation value J is greater than a given threshold $V_{jmax}$, the association is canceled. In this case, the region corresponding to the region $R_{t1,m}$ is regarded as being not observed at the time $t_2$, and the longitudinal parameter measurement on the region $R_{t1,m}$ is aborted at the time $t_1$. The modification is effective for reducing the influence of noises.
According to a second modification, the distance between the centroids in the region $R_{t2,n^}$ corresponding to the region $R_{t1,m}$ is determined, and if the distance between the centroids is greater than a given threshold $V_{dmax}$, the association is canceled. In this case, the region corresponding to the region $R_{t1,m}$ is regarded as being not observed at the time $t_2$, and the longitudinal parameter measurement on the region $R_{t1,m}$ is aborted at the time $t_1$. The modification is effective for reducing errors in the association between the cellular regions.
With the above procedures, the cell parameters are longitudinally measured.

In the subsequent step S7, viability of respective cells is determined by the cell viability determination unit 313. The determination of cell viability in the cell viability determination unit 313 includes the following plurality of procedures, and one or more procedures are used for the determination.

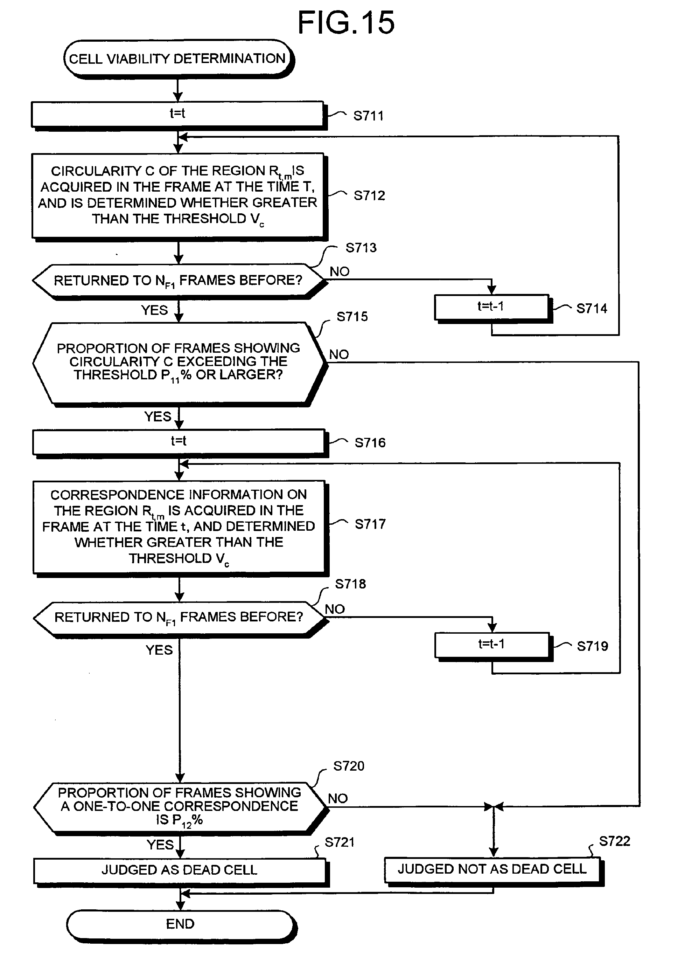

FIG. 15 is a schematic flowchart showing the first procedure of cell viability determination. The schematic flowchart shown in FIG. 15 shows an example of the procedure of cell viability determination on the basis of the cell property that, when a cell die during culture without undergoing physical damages, it has a generally circular shape, and ceases its activity while maintaining the generally circular shape.

In the first place, while going back one by one from the frame containing the cellular region $R_{t,m}$ at the time t to the frame $N_{F1}$ (a given number) frames earlier, the circularity C of the cellular region $R_{t,m}$ in the frames photographed at different time points is acquired as a cell parameter, the acquired circularity C is compared with the given threshold $V_C$, and whether the circularity C is greater than the threshold $V_C$ is repeatedly determined for all frames (steps S711 to S714). The circularity C is defined by $C = 4\pi A/L^2$, wherein A is the area of the cellular region $R_{t,m}$, and L is the length (perimeter) of the contour, and used as a scale for determining the degree of the roundness of the cell shape.

Subsequently, with regard to the plurality of frames from the frame at the time t to the frame $N_{F1}$ frames earlier, whether the proportion of the frames showing a circularity C exceeding the threshold $V_C$ is the given threshold $P_{11}$% or larger is determined (step 5715). When the proportion of the frames with a circularity C exceeding the threshold $V_C$ is the given threshold $P_{11}$ or larger (step S715: Yes), while going back one by one from the frame containing the cellular region $R_{t,m}$ at the time t to the frame $N_{F1}$ (a given number) frames earlier, the cellular region $R_{t,m}$ is correlated with those in the frames within the period, and whether the correspondence is one-to-one or not is repeatedly determined for all frames (steps S716 to S719). With regard to the frames in the period, when the proportion of frames showing a one-to-one correspondence is the given threshold $P_{12}$% or larger (step S720: Yes), the cellular region $R_{t,m}$ is judged as being dead (step S721). The reason is that the cellular regions at different time points, which are regarded as the same region, are recognized as maintaining a round state brought about by cell death.

On the other hand, when the proportion of the frames with a circularity C exceeding the threshold $V_C$ is less than the given threshold $P_{11}$% (step S715: No), the cellular region $R_{t,m}$ is judged as not being dead (step S722). The reason is that the generally circular shape characteristic of a dead cell is not observed. The given threshold $P_{11}$% may be set at 100%, where cell death is denied when the circularity C is not greater than the threshold $V_C$ in all the frames. However, the threshold $P_{11}$% is not necessarily required to be 100%, and cell death may be denied when the proportion falls below the threshold $P_{11}$ set at below 100%. The reason is that acquisition of cell parameters, or circularity herein, may be inappropriate in some frames. As a result of this, false detection of cell death is prevented.

Among the frames from the frame $N_{F1}$ frames earlier, when the proportion of the frames not showing a one-to-one correspondence is the given threshold $P_{12}$% or larger (step S720: No), the cellular region $R_{t,m}$ is judged as not being dead (step S722). A cell shows a generally circular shape not only when it is dead but also when it is in the mitotic phase (M phase) in an average cell cycle. However, a cell in the mitotic phase can be distinguished from a dead cell because the mitotic phase is followed immediately by cell division, so that the correspondence between the cellular regions at different time points becomes one to plural, and thus the proportion of the frames showing a one-to-one correspondence falls below the given threshold $P_{12}$%, while the proportion of the frames in which a plurality of cellular regions are correlated with one cellular region increases. On this account, the number $N_{F1}$ defining the number of frames corresponding to the lapse of time is desirably set at a value such that the imaging period from the $N_{F1}$ frames before is longer than the mitotic phase in the average cell cycle of the cell under observation. As in the case of the threshold $P_{11}$%, the given threshold $P_{12}$% may be 100%, but is not necessarily required to be 100%

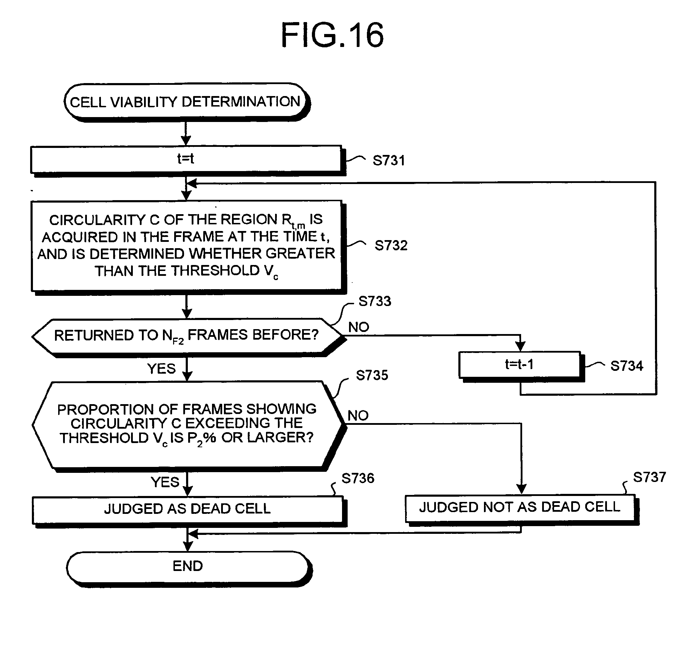

FIG. 16 is a schematic flowchart showing the second procedure of cell viability determination. In the same manner as the first procedure, the schematic flowchart shown in FIG. 16 shows an example of the procedure of cell viability determination on the basis of the cell property that, when a cell dies during culture without undergoing physical damages, it has a generally circular shape, and ceases its activity while maintaining the generally circular shape.

In the first place, while going back one by one from the frame containing the cellular region $R_{t,m}$ at the time t to the frame $N_{F2}$ (a given number) frames earlier, the circularity C of the cellular region $R_{t,m}$ in the frames photographed at different time points is acquired as a cell parameter, the acquired circularity C is compared with a given threshold $V_C$, and whether the circularity C is greater than the threshold $V_C$ is repeatedly determined for all frames (steps S731 to S734). The definition of the circularity C is as described in the first procedure. With regard to the plurality of frames from the frame at the time t to the frame $N_{F2}$ frames earlier, when the proportion of the frames showing a circularity C exceeding the threshold $V_C$ is the given threshold $P_2$ or larger (step S735: Yes), the cellular region $R_{t,m}$ is judged as being dead (step S736). The reason is that the cellular region is recognized as maintaining a round state brought about by cell death.

On the other hand, when the proportion of the frames with a circularity C exceeding the threshold $V_C$ is less than the given threshold $P_2$% (step S735: No), the cellular region $R_{t,m}$ is judged as not being dead (step S737). The reason is that the generally circular shape characteristic of a dead cell is not observed. A cell shows a generally circular shape not only when it is dead but also when it is in the mitotic phase (M phase) in an average cell cycle. However, a cell in the mitotic phase can be distinguished from a dead cell because a cell in the mitotic phase changes in its shape depending on the cell activity after the mitotic phase, and does not maintain the generally circular shape. On this account, the number $N_{F2}$ defining the number of frames corresponding to the lapse of time is desirably set at a value such that the imaging period from the $N_{F2}$ frames before is longer than the mitotic phase in the average cell cycle of the cell under observation. As in the case of the threshold $P_{11}\%$, the given threshold $P_2\%$ may be 100%, but is not necessarily required to be 100%.

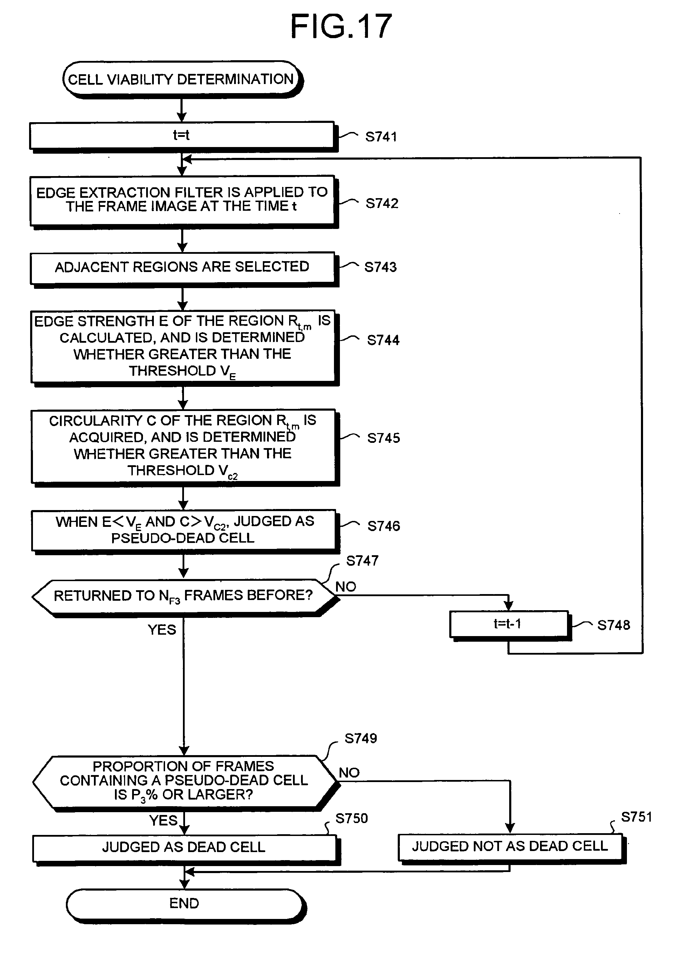

FIG. 17 is a schematic flowchart showing the third procedure of the cell viability determination. The schematic flowchart shown in FIG. 17 represents a process of detecting a dead cell, specifically one floating in the culture medium A. It represents an example of the procedure of cell viability determination on the basis of the property that a dead cell detached from the slide glass 102 has a generally circular shape as in the case of the above procedure, and shows a blurred image because the cell lies before the focus position for imaging (the surface of the slide glass 102), so that the edge strength decreases in the contour areas. More specifically, in place of the shape property of circularity, the cell position (depth of focus) to the surface of the slide glass 102 is comprehensively determined using the edge strength as a cell parameter thereby detecting cell detachment brought about by cell death.

In the first place, the frame image containing the cellular region $R_{t,m}$ at the time t is acquired, and the above-described output result of the low-pass filter of edge preservation type is subjected to a general edge extraction filter, for example a Sobel filter, to obtain an edge image (steps S741 and S742). Subsequently, two arbitrary cellular regions adjacent to the cellular region $R_{t,m}$ contained in the frame at the time t are selected in consideration of the borderline between them (step S743), the edge strength E defined by Formula (6) is determined as a cell parameter, and whether the edge strength E is greater than the given threshold $V_E$ is judged (step S744).

[Formula 6]

$$E = \sum_P E(P) \qquad\qquad (6)$$

Here, E(P) is the intensity of the pixel P in the edge image, and $\Sigma$ represents the integration of all the pixels on the contours between the cellular regions.

In the same manner, the circularity C of the cellular region $R_{t,m}$ is acquired, and whether the circularity C is greater than the given threshold $V_{C2}$ is determined (step 5745). When the circularity C of the cellular region $R_{t,m}$ is greater than the given threshold $V_{C2}$ and the edge strength E is less than the given threshold $V_E$, the cell is judged as pseudo-dead (step 5746). While going back from the frame containing the target cellular region $R_{t,m}$ at the time t to the frame $N_{F3}$ (a given number) frames earlier, the determination of pseudo-cell death is repeatedly carried out for all frames (steps S747, S748, S742 to 5746). When the proportion of the frames containing a pseudo-dead cell is equal to or greater than the given threshold $P_3\%$ (step S749: Yes), the cell is judged as being dead (step S750). On the other hand, when the proportion of the frames containing a pseudo-dead cell is less than the given threshold $P_3\%$ (step S749: No), the cell is judged as not being dead (step S751).

In the third procedure, the number $N_{F3}$ defining the number of frames corresponding to the lapse of time is desirably set at a value such that the imaging period from the $N_{F3}$ frames before is longer than the mitotic phase in the average cell cycle of the cell under observation. As in the case of the threshold $P_{11}\%$, the given threshold $P_3\%$ may be 100%, but is not necessarily required to be 100%.

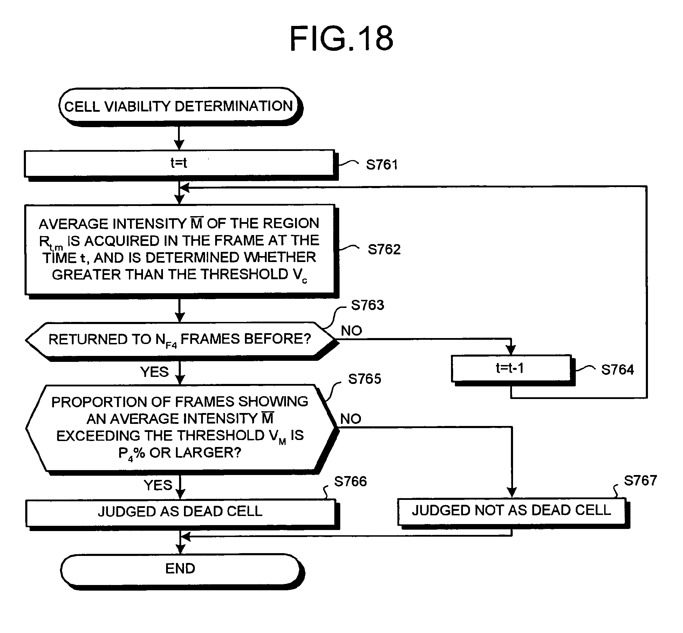

FIG. 18 is a schematic flowchart showing the fourth procedure of the cell viability determination. The schematic flowchart shown in FIG. 18 represents an example of the procedure of cell viability determination on the basis of the cell property that, when a cell dies during culture without undergoing physical damages, it is rounded while shrunk due to surface tension of the cell membrane, and ceases its activity in a shrunk state. The shrinkage decreases the apparent area, which results in a higher average intensity than that of a living cell. Accordingly, the event of shrinkage is evaluated in terms of the average intensity.

In the first place, while going back one by one from the frame containing the cellular region $R_{t,m}$ at the time t to the frame $N_{F2}$ (a given number) frames earlier, the average intensity (/M) of the cellular region $R_{t,m}$ in the frames photographed at different time points is acquired as a cell parameter, the acquired average intensity (/M) is compared with the given threshold $V_M$, and whether the average intensity (/M) is greater than the threshold $V_M$ is repeatedly determined for all frames (steps S761 to S764). With regard to the plurality of frames from the frame at the time t to the frame $N_{F4}$ frames earlier, when the proportion of the frames showing an average intensity (/M) C exceeding the threshold $V_M$ is greater than the given threshold $P_4\%$ (step S765: Yes), the cellular region $R_{t,m}$ is judged as being dead (step S766). The reason is that the cellular region is recognized as maintaining a round and bright state brought about by cell death.

On the other hand, when the proportion of the frames showing an average intensity (/M) exceeding the threshold

$V_M$ is less than the given threshold $P_4$% (step S765: No), the cellular region $R_{t,m}$ is judged as not being dead (step S767). The reason is that the round and bright state characteristic of a dead cell is not observed. More specifically, the temporal increase in the cell intensity is observed not only in a dead cell but also in a cell in the mitotic phase (M phase) in an average cell cycle. However, a cell in the mitotic phase can be distinguished from a dead cell because a cell in the mitotic phase decreases in its intensity depending on the cell activity after the mitotic phase, and does not maintain a bright state. On this account, the number $N_{F4}$ defining the number of frames corresponding to the lapse of time is desirably set at a value such that the imaging period from the $N_{F4}$ frames before is longer than the mitotic phase in the average cell cycle of the cell under observation. As in the case of the threshold $P_{11}$%, the given threshold $P_4$% may be 100%, but is not necessarily required to be 100%.

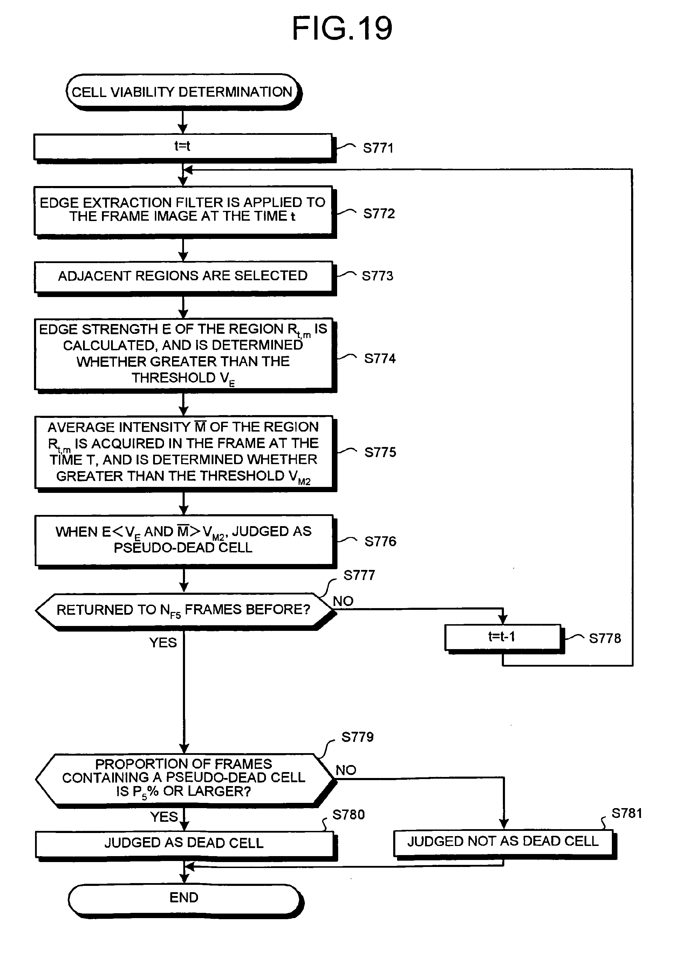

FIG. 19 is a schematic flowchart showing the fifth procedure of the cell viability determination. The schematic flowchart shown in FIG. 19 represents a process of detecting a dead cell, specifically one floating in the culture medium A. It represents an example of the procedure of cell viability determination on the basis of the property that, as in the case of the above-described third procedure, a dead cell detached from the slide glass 102 exhibits a higher intensity and provides a blurred image because the cell lies before the focus position for imaging (the surface of the slide glass 102), so that the edge strength decreases in the contour areas.

In the first place, the frame image containing the cellular region $R_{t,m}$ at the time t is acquired, and the above-described output result of the low-pass filter of edge preservation type is subjected to a general edge extraction filter, for example a Sobel filter, to obtain an edge image (steps S771 and S772). Subsequently, two arbitrary cellular regions adjacent to the cellular region $R_{t,m}$ contained in the frame at the time t are selected in consideration of the borderline between them (step S773), the edge strength E defined by the above-described Formula (6) is determined as a cell parameter, and whether the edge strength E is greater than the given threshold $V_E$ is judged (step 5774).

In the same manner, the average intensity (/M) of the cellular region $R_{t,m}$ is acquired, and whether the average intensity (/M) is greater than the given threshold $V_{M2}$ is determined (step S775). When the average intensity (/M) of the cellular region $R_{t,m}$ is greater than the given threshold $V_{M2}$ and the edge strength E is less than the given threshold $V_E$, the cell is judged as pseudo-dead (step 5776). While going back from the frame containing the target cellular region $R_{t,m}$ at the time t to the frame a given number of frames $N_{F5}$ earlier, the determination of pseudo-cell death is repeatedly carried out for all the frames (steps S777, S778, S772 to S776). When the proportion of the frames containing a pseudo-dead cell is equal to or greater than the given threshold $P_5$% (step S779: Yes), the cell is judged as being dead (step 5780). On the other hand, when the proportion of the frames containing a pseudo-cell death is less than the given threshold $P_5$% (step S779: No), the cell is judged as not being dead (step 5781).

In the fifth procedure, the number $N_{F5}$ defining the number of frames corresponding to the lapse of time is desirably set at a value such that the imaging period from the $N_{F5}$ frames before is longer than the mitotic phase in the average cell cycle of the cell under observation. As in the case of the threshold $P_{11}$%, the given threshold $P_5$% may be 100%, but is not necessarily required to be 100%.

In the first to fifth procedures of the cell viability determination, a cell judged as not dead is regarded as being alive.

The cell viability determination may employ any of the first to fifth procedures. In addition, determination results obtained through two or more of the procedures may be combined thereby improving the accuracy of the determination.

The cell viability determination method according to the present embodiment is capable of determining cell viability for respective cells, so that the number of living cells and dead cells can be accurately known, and the survival rate of the cells during culture can be accurately determined. In addition, cell viability is determined under measurement of a cell parameter in respective cells, so that the dying process of a cell during long-term culture can be accurately reproduced with data. On this account, cell viability is accurately determined with minimum damages to the cells during culture and without requiring the introduction of a special dye or gene.

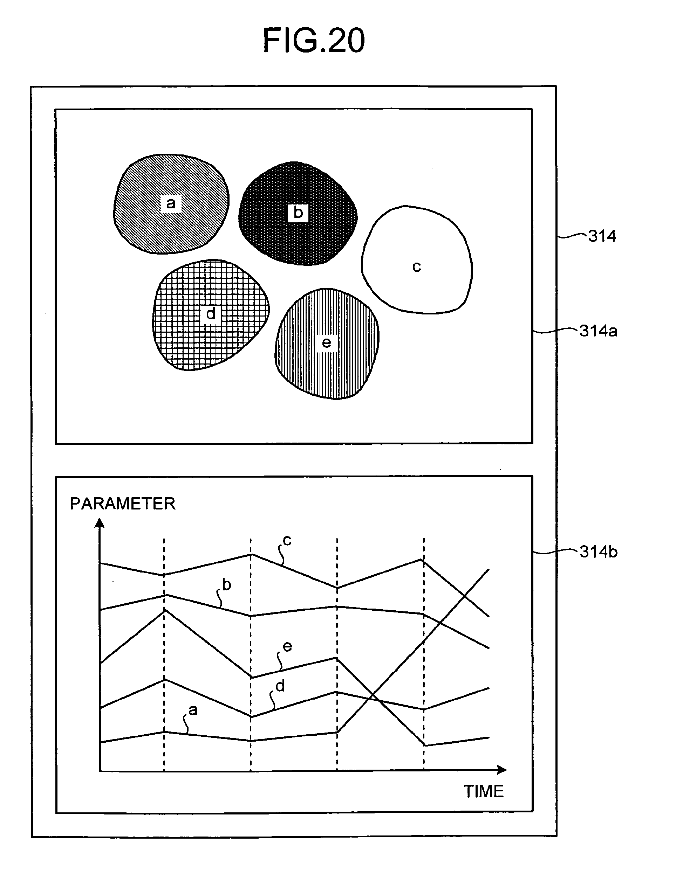

Finally, as the processing in step S8 shown in FIG. 9, the recognized cellular region and measured cell parameters are displayed on the display unit 304 as a means for displaying cell parameters. FIG. 20 is an explanatory drawing showing a display example of a processing result. The display screen 314 composing the display unit 304 has two display regions 314a and 314b, and the display region 314a displays the cellular regions recognized at the time points of processing. The cellular regions is subjected to labeling processing for giving the regions identifiable colors, intensities, line types, and patterns, and displayed as, for example, label images a to e. A label image may be displayed in synchronization with an infrared image or a fluorescence image in the same display range, or two or more of a label image, an infrared image, and a fluorescence image may be overlapped each other. Alternatively, superimpose display may be carried out. The measured cell parameters are displayed in the display region 314b in the form of a line chart with the horizontal axis as time and the vertical axis as parameter values.

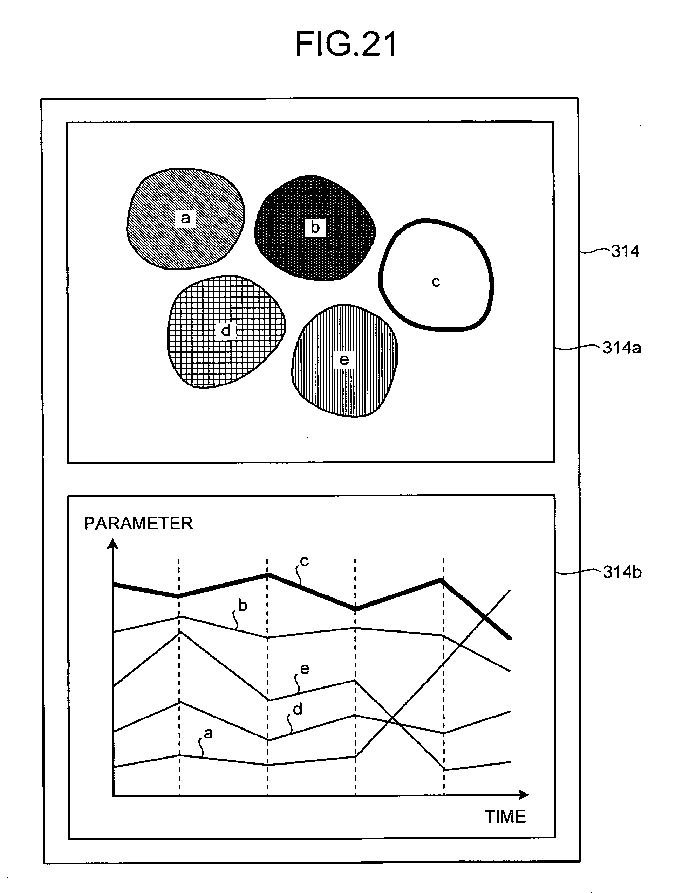

In addition, visibility of the display contents can be improved by highlighting the display contents in synchronism with each other according to the operator's mouse-operation or the like through the input unit 303. FIG. 21 shows an explanatory drawing showing an example of highlighting, in which, for example, when a label image c is selected as the object of highlighting, the line chart of the corresponding cell parameter is also highlighted. In this case, when the operator selectively highlights one of them, the corresponding other one is also highlighted in synchronization. At this time, a cell judged as being dead is highlighted in a visually recognizable manner. For example, the cell may be blinked or indicated with a diagram or character different in color, intensity, shape, or pattern. Alternatively, the corresponding cell may be not displayed, or these displays may be switched.

According to the present embodiment, the living cells C loaded with a fluorescent protein are observed. The fluorescent protein may be replaced with a luminescence gene such as a luciferase gene thereby acquiring a luminescence image in place of a fluorescence image. In this case, the excitation lighting unit 203 and dichroic mirror 204 are unnecessary, so that the structure is simplified. The luminescence image is acquired by the fluorescence imaging unit 207 as an optical cell imaging means. The luminescence image may be processed in the same manner as for a fluorescence image. Accordingly, cell image data can be acquired for cell observation even in the cases where the cell emits light by itself or light other than infrared light such as fluorescence.

The fluorescent protein used in the present embodiment is expressed with no localization in the cells, but the fluorescent protein may be expressed with being localized in the nucleus, cytoplasm, nuclear membrane, cell membrane, or organelle.

The cell parameters measured in the parameter measurement unit 307 are not limited to those listed in the present embodiment, and may include any one or more of the area, perimeter, bounding rectangle position, X-way Feret's diameter, Y-way Feret's diameter, minimum Feret's diameter, maximum Feret's diameter, average Feret's diameter, convex perimeter, circularity (roundness), number of holes, roughness (ratio of convex perimeter to perimeter), Euler number, length, width, aspect ratio, total intensity, minimum intensity, maximum intensity, average intensity, standard deviation of the intensity, variance of intensity, entropy, position of centroid, secondary moment, and direction of the principle axis.

In addition, the parameter measurement unit 307 may request to a group composed of a plurality of arbitrary cells for one or more of factors including the number of cells, minimum intercellular distance, maximum intercellular distance, average intercellular distance, standard deviation of intercellular distance, variance of intercellular distance, and the minimum value, maximum value, average, standard deviation, difference, total, and intermediate value of the measured parameters of respective cells.

As described above, according to the present embodiment, there is provided an apparatus for accurately determining cell viability with minimum damages to the cells and without requiring the introduction of a special dye or gene, wherein the apparatus images a plurality of living cells labeled with a fluorescence substance over a period of time, recognizes respective cells, and tracks the change of position over time.

The present invention is not limited to the above-described embodiment, and may be variously modified without departing from the scope of the present invention. For example, the above-described procedure by the cell recognition unit 306, parameter measurement unit 307, cell tracking unit 308, cell viability determination unit 313, and other units may be carried out by executing a previously prepared cell observation program on a microcomputer such as the control unit 301. The cell observation program may be distributed through a network such as the internet. In addition, the cell observation program may be stored in a microcomputer-readable recording medium such as a hard disk, FD, CD-ROM, MO, or DVD and retrieved from the recording medium by a microcomputer for execution.

INDUSTRIAL APPLICABILITY

As described above, the cell observation apparatus, cell observation method, and cell observation program according to the present invention are useful in longitudinal observation of living cells during long-term culture, and particularly suitable for determination of cell viability during culture.

**Claims**

1. A cell observation apparatus comprising:

   a cell recognition unit that recognizes a cellular region representing a cell from cell image data acquired by imaging the cell at a plurality of time points;
   a cell parameter measurement unit that measures cell parameters characteristic of the cellular region recognized by the cell recognition unit; and
   a cell viability determination unit that compares the cell parameters measured by the cell parameter measurement unit with thresholds thereby determining cell viability.

2. The cell observation apparatus according to claim 1, further comprising a cell tracking unit that interrelates the cellular regions recognized from the cell image data acquired at different time points, wherein the cell viability determination unit determines the cell viability by repeatedly comparing the cell parameters with the thresholds on the cellular regions interrelated by the cell tracking unit.

3. The cell observation apparatus according to claim 2, wherein the cell viability determination unit judges the cell represented by the cellular regions interrelated by the cell tracking unit as not being dead when the same conclusion is derived from the results of the repeated comparisons at a rate below a given rate.

4. The cell observation apparatus according to claim 2 or 3, wherein the cell viability determination unit judges the cell represented by the cellular regions as not being dead when two or more of the cellular regions are correlated with one of the cellular regions by the cell tracking unit.

5. The cell observation apparatus according to any one of claims 1 to 4, wherein the cell viability determination unit determines viability of the cell by repeatedly comparing the cell parameters with thresholds, the cell parameters being measured in the cellular regions at a plurality of time points in the imaging period established so as to exceed the mitotic phase in the average cell cycle of the cell under observation.

6. The cell observation apparatus according to any one of claims 1 to 5, wherein the cell parameters include at least a circularity of the cellular regions.

7. The cell observation apparatus according to any one of claims 1 to 5, wherein the cell parameters include at least an average intensity of the cellular regions.

8. The cell observation apparatus according to claim 6 or 7, wherein the cell parameters include at least an edge strength of the cellular regions.

9. The cell observation apparatus according to any one of claims 1 to 8, further comprising a culture unit that cultures a cell.

10. The cell observation apparatus according to any one of claims 1 to 9, wherein the cell is loaded with a fluorescent protein.

11. The cell observation apparatus according to claim 10, wherein the cell is loaded with a fluorescent protein which is expressed with no localization.

12. The cell observation apparatus according to any one of claims 1 to 11, further comprising an imaging unit that acquires images of the cell at a plurality of time points.

13. A cell observation program for observing a cell in a cell observation apparatus, the cell observation program causes cell observation apparatus to perform:

a cell recognition step of recognizing a cellular region representing a cell from cell image data acquired by imaging the cell at a plurality of time points;
a cell parameter measurement step of measuring cell parameters characteristic of the cellular region recognized by the cell recognition step; and
a cell viability determination step of comparing the cell parameters measured by the cell parameter measurement step with thresholds thereby determining cell viability.

14. The cell observation program according to claim 13, further comprising a cell tracking step of interrelating the cellular regions recognized from the cell image data acquired at different time points, wherein the cell viability determination step determines the cell viability by repeatedly comparing the cell parameters with the thresholds on the cellular regions interrelated by the cell tracking step.

15. The cell observation program according to claim 14, wherein the cell viability determination step judges the cell represented by the cellular regions interrelated by the cell tracking step as not being dead when the same conclusion is derived from the results of the repeated comparisons at a rate below a given rate.

16. The cell observation program according to claim 14 or 15, wherein the cell viability determination step judges the cell represented by the cellular regions as not being dead when two or more of the cellular regions are correlated with one of the cellular regions by the cell tracking step.

17. The cell observation program according to any one of claims 13 to 16, wherein the cell viability determination step determines viability of the cell by repeatedly comparing the cell parameters with thresholds, the cell parameters being measured in the cellular regions at a plurality of time points in the imaging period established so as to exceed the mitotic phase in the average cell cycle of the cell under observation.

18. The cell observation program according to any one of claims 13 to 17, wherein the cell parameters include at least a circularity of the cellular regions.

19. The cell observation program according to any one of claims 13 to 17, wherein the cell parameters include at least an average intensity of the cellular regions.

20. The cell observation program according to claim 18 or 19, wherein the cell parameters include at least an edge strength of the cellular regions.

21. A cell observation method for observing a cell in a cell observation apparatus that includes a culture unit which cultures the cell and an imaging unit which images the cell contained in the culture unit, the method comprising:

a cell recognition step of recognizing a cellular region representing the cell from cell image data acquired by imaging, by the imaging unit, the cell cultured in the culture unit at a plurality of time points;
a cell parameter measurement step of measuring cell parameters characteristic of the cellular region recognized by the cell recognition unit; and
a cell viability determination step of comparing the cell parameters measured by the cell parameter measurement unit with thresholds thereby determining cell viability.

22. The cell observation method according to claim 21, further comprising a cell tracking step of interrelating the cellular regions recognized from the cell image data acquired at different time points, wherein the cell viability determination step determines the cell viability by repeatedly comparing the cell parameters with the thresholds on the cellular regions interrelated by the cell tracking step.

# FIG.1

# FIG.2

# FIG.3

# FIG.4

108a

108

# FIG.5

112

111

202

# FIG.6

# FIG.7

# FIG.8

EP 1 881 061 A1

$t_{k-1}$        $t_k$      TIME

VISUAL FIELD 1 — IMAGING (k-1)TH     IMAGING K TH

VISUAL FIELD 2 — IMAGING (k-1)TH     IMAGING K TH

VISUAL FIELD 3 — IMAGING (k-1)TH     IMAGING K TH

VISUAL FIELD N — IMAGING (k-1)TH     IMAGING K TH

# FIG.9

START

S1 IMAGING

S2 PREPROCESSING

S3 CELL RECOGNITION

S4 PARAMETER MEASUREMENT

S5 CELL TRACKING

S6 CORRECTION OF TRACKING RESULT

S7 CELL VIABILITY DETERMINATION

S8 RESULT DISPLAY

S9 IS OBSERVE COMPLETED?

NO

YES

END

# FIG.10

| -1 | -1 | -1 |
|----|----|----|
| -1 | 9 | -1 |
| -1 | -1 | -1 |

# FIG.11

START

EXTRACTION OF PEAK INTENSITY — S311

SELECTION OF ADJACENT REGIONS — S312

CALCULATION OF DISTANCE $D_{UW}$ — S313

$D_{UW} \leqq V_{UW}$? — S314

YES

INTEGRATION OF CELLULAR REGIONS — S315

NO

ARE ALL COMBINATIONS PROCESSED? — S316

NO

YES

END

# FIG.12

```
        ┌─────────────┐
        │    START    │
        └─────────────┘
               │
               ▼
   ┌──────────────────────────┐
   │  APPLICATION OF EDGE     │  S321
   │  EXTRACTION FILTER       │
   └──────────────────────────┘
               │
               ▼
   ┌──────────────────────────┐
   │  SELECTION OF ADJACENT   │  S322
   │  REGIONS                 │
   └──────────────────────────┘
               │
               ▼
   ┌──────────────────────────┐
   │  CALCULATION OF EDGE     │  S323
   │  STRENGTH D_UE           │
   └──────────────────────────┘
               │
               ▼
            S324
        ╱─────────────╲      YES        S325
       ╱  D_UE ≦ V_UE? ╲───────────┐  ┌────────────────────┐
       ╲               ╱           └─▶│  INTEGRATION OF    │
        ╲─────────────╱               │  CELLULAR REGIONS  │
               │ NO                   └────────────────────┘
               ▼◀──────────────────────────────┘
            S326
        ╱─────────────╲     NO
       ╱   ARE ALL     ╲──────────┐
       ╲  COMBINATIONS ╱          │
        ╲ PROCESSED?  ╱           │
         ╲───────────╱            │
               │ YES              │
               ▼                  │
        ┌─────────────┐           │
        │     END     │           │
        └─────────────┘           │
```

$D_{UE} \leqq V_{UE}$?

$D_{UE}$

# FIG.13

| | POSITION OF CENTROID | AREA | CIRCULARITY | TOTAL INTENSITY | AVERAGE INTENSITY | STANDARD DEVIATION OF INTENSITY |
|---|---|---|---|---|---|---|
| CELLULAR REGION $Rt_{1,1}$ | $X_1, Y_1$ | $A_1$ | $C_1$ | $S_1$ | $MV_1$ | $SD_1$ |
| CELLULAR REGION $Rt_{1,2}$ | $X_2, Y_2$ | $A_2$ | $C_2$ | $S_2$ | $MV_2$ | $SD_2$ |
| : | : | : | : | : | : | : |
| CELLULAR REGION $Rt_{1,m}$ | $X_m, Y_m$ | $A_m$ | $C_m$ | $S_m$ | $MV_m$ | $SD_m$ |
| : | : | : | : | : | : | : |
| CELLULAR REGION $Rt_{1,M}$ | $X_M, Y_M$ | $A_M$ | $C_M$ | $S_M$ | $MV_M$ | $SD_M$ |

# FIG.14

| | CELLULAR REGION $Rt_{2,1}$ | CELLULAR REGION $Rt_{2,2}$ | | CELLULAR REGION $Rt_{2,n}$ | | CELLULAR REGION $Rt_{2,N}$ |
|---|---|---|---|---|---|---|
| CELLULAR REGION $Rt_{1,1}$ | $J_{1,1}$ | $J_{1,2}$ | ... | $J_{1,n}$ | ... | $J_{1,N}$ |
| CELLULAR REGION $Rt_{1,2}$ | $J_{2,1}$ | $J_{2,2}$ | ... | $J_{2,n}$ | ... | $J_{2,N}$ |
| : | : | : | | : | | : |
| CELLULAR REGION $Rt_{1,m}$ | $J_{m,1}$ | $J_{m,2}$ | ... | $J_{m,n}$ | ... | $J_{m,N}$ |
| : | : | : | | : | | : |
| CELLULAR REGION $Rt_{1,M}$ | $J_{M,1}$ | $J_{M,2}$ | ... | $J_{M,n}$ | ... | $J_{M,N}$ |

# FIG.15

```
       ( CELL VIABILITY DETERMINATION )
                    │
                    ▼
       ┌─────────────────────────────┐
       │            t=t              │────  S711
       └─────────────────────────────┘
                    │
                    ▼
       ┌─────────────────────────────┐
       │ CIRCULARITY C OF THE REGION │
       │ R_{t,m} IS ACQUIRED IN THE  │
       │ FRAME AT THE TIME T,        │──  S712
       │ AND IS DETERMINED WHETHER   │
       │ GREATER THAN THE THRESHOLD  │
       │ V_c                         │
       └─────────────────────────────┘
                    │          S713
                    ▼
       ╱─────────────────────────╲   NO    ┌──────────┐
       ⟨ RETURNED TO N_{F1} FRAMES ⟩─────── │  t=t-1   │── S714
       ╲    BEFORE?                ╱         └──────────┘
                    │ YES
                    ▼                S715
       ╱─────────────────────────╲   NO
       ⟨ PROPORTION OF FRAMES      ⟩────────
       ⟨ SHOWING CIRCULARITY C     ⟩
       ⟨ EXCEEDING THE THRESHOLD   ⟩
       ⟨ P_{11}% OR LARGER?        ⟩
       ╲─────────────────────────╱
                    │ YES
                    ▼
       ┌─────────────────────────────┐
       │            t=t              │──  S716
       └─────────────────────────────┘
                    │
                    ▼
       ┌─────────────────────────────┐
       │ CORRESPONDENCE INFORMATION  │
       │ ON THE REGION R_{t,m} IS    │
       │ ACQUIRED IN THE FRAME AT    │──  S717
       │ THE TIME t, AND DETERMINED  │
       │ WHETHER GREATER THAN THE    │
       │ THRESHOLD V_c               │
       └─────────────────────────────┘
                    │          S718
                    ▼
       ╱─────────────────────────╲   NO    ┌──────────┐
       ⟨ RETURNED TO N_{F1} FRAMES ⟩─────── │  t=t-1   │── S719
       ╲    BEFORE?                ╱         └──────────┘
                    │ YES
                    ▼                S720
       ╱─────────────────────────╲   NO
       ⟨ PROPORTION OF FRAMES      ⟩────────
       ⟨ SHOWING A ONE-TO-ONE      ⟩
       ⟨ CORRESPONDENCE IS P_{12}% ⟩
       ╲─────────────────────────╱
                    │ YES            S721              S722
                    ▼                                   
       ┌─────────────────────────────┐   ┌──────────────────────────┐
       │     JUDGED AS DEAD CELL     │   │ JUDGED NOT AS DEAD CELL  │
       └─────────────────────────────┘   └──────────────────────────┘
                    │                                   │
                    ▼                                   
              (     END     )◄──────────────────────────┘
```

# FIG.16

```
┌────────────────────────────────┐
│  CELL VIABILITY DETERMINATION  │
└────────────────────────────────┘
                 │
                 ▼
┌────────────────────────────────┐
│              t=t               │────── S731
└────────────────────────────────┘
                 │
                 ▼
┌────────────────────────────────┐
│ CIRCULARITY C OF THE REGION R_{t,m} IS │
│ ACQUIRED IN THE FRAME AT THE TIME t,   │──── S732
│ AND IS DETERMINED WHETHER GREATER      │
│      THAN THE THRESHOLD V_c            │
└────────────────────────────────┘
                 │
                 ▼          ┌─ S733
    RETURNED TO N_{F2} FRAMES BEFORE?      NO ──────┐
                 │                                  │
                YES                          ┌──────────────┐
                 ▼          ┌─ S735          │    t=t-1     │── S734
    PROPORTION OF FRAMES SHOWING            └──────────────┘
    CIRCULARITY C EXCEEDING THE        NO
    THRESHOLD V_c IS P_2% OR LARGER? ──────────┐
                 │                             │
                YES        ┌─ S736             ▼           ┌─ S737
    ┌──────────────────────┐        ┌──────────────────────────┐
    │  JUDGED AS DEAD CELL │        │ JUDGED NOT AS DEAD CELL  │
    └──────────────────────┘        └──────────────────────────┘
                 │                             │
                 ▼◄────────────────────────────┘
         ┌──────────────┐
         │     END      │
         └──────────────┘
```

# FIG.17

```
      ( CELL VIABILITY DETERMINATION )
                    │
                    ▼
      ┌─────────────────────────────┐
      │            t=t              │───  S741
      └─────────────────────────────┘
                    │
                    ▼
      ┌─────────────────────────────┐
      │ EDGE EXTRACTION FILTER IS   │
      │   APPLIED TO THE FRAME      │───  S742
      │     IMAGE AT THE TIME t     │
      └─────────────────────────────┘
                    │
                    ▼
      ┌─────────────────────────────┐
      │ ADJACENT REGIONS ARE        │───  S743
      │         SELECTED            │
      └─────────────────────────────┘
                    │
                    ▼
      ┌─────────────────────────────┐
      │ EDGE STRENGTH E OF THE      │
      │ REGION Rt,m IS CALCULATED,  │───  S744
      │ AND IS DETERMINED WHETHER   │
      │ GREATER THAN THE THRESHOLD  │
      │          VE                 │
      └─────────────────────────────┘
                    │
                    ▼
      ┌─────────────────────────────┐
      │ CIRCULARITY C OF THE        │
      │ REGION Rt,m IS ACQUIRED,    │───  S745
      │ AND IS DETERMINED WHETHER   │
      │ GREATER THAN THE THRESHOLD  │
      │          Vc2                │
      └─────────────────────────────┘
```

EDGE STRENGTH E OF THE REGION $R_{t,m}$ IS CALCULATED, AND IS DETERMINED WHETHER GREATER THAN THE THRESHOLD $V_E$ — S744

CIRCULARITY C OF THE REGION $R_{t,m}$ IS ACQUIRED, AND IS DETERMINED WHETHER GREATER THAN THE THRESHOLD $V_{c2}$ — S745

WHEN $E < V_E$ AND $C > V_{C2}$, JUDGED AS PSEUDO-DEAD CELL — S746

RETURNED TO $N_{F3}$ FRAMES BEFORE? — S747

NO → t=t-1 — S748

YES

PROPORTION OF FRAMES CONTAINING A PSEUDO-DEAD CELL IS $P_3$% OR LARGER? — S749

NO

YES → JUDGED AS DEAD CELL — S750

JUDGED NOT AS DEAD CELL — S751

END

29

# FIG.18

CELL VIABILITY DETERMINATION

$t=t$ — S761

AVERAGE INTENSITY $\overline{M}$ OF THE REGION $R_{t,m}$ IS ACQUIRED IN THE FRAME AT THE TIME $t$, AND IS DETERMINED WHETHER GREATER THAN THE THRESHOLD $V_c$ — S762

RETURNED TO $N_{F4}$ FRAMES BEFORE? — S763

NO → $t=t-1$ — S764

YES ↓

PROPORTION OF FRAMES SHOWING AN AVERAGE INTENSITY $\overline{M}$ EXCEEDING THE THRESHOLD $V_M$ IS $P_4\%$ OR LARGER? — S765

NO → JUDGED NOT AS DEAD CELL — S767

YES ↓

JUDGED AS DEAD CELL — S766

END

# FIG.19

CELL VIABILITY DETERMINATION

t=t — S771

EDGE EXTRACTION FILTER IS APPLIED TO THE FRAME IMAGE AT THE TIME t — S772

ADJACENT REGIONS ARE SELECTED — S773

EDGE STRENGTH E OF THE REGION $R_{t,m}$ IS CALCULATED, AND IS DETERMINED WHETHER GREATER THAN THE THRESHOLD $V_E$ — S774

AVERAGE INTENSITY $\overline{M}$ OF THE REGION $R_{t,m}$ IS ACQUIRED IN THE FRAME AT THE TIME T, AND IS DETERMINED WHETHER GREATER THAN THE THRESHOLD $V_{M2}$ — S775

WHEN $E < V_E$ AND $\overline{M} > V_{M2}$, JUDGED AS PSEUDO-DEAD CELL — S776

S777
RETURNED TO $N_{F5}$ FRAMES BEFORE? — NO

YES

t=t-1 — S778

S779
PROPORTION OF FRAMES CONTAINING A PSEUDO-DEAD CELL IS $P_5$% OR LARGER? — NO

YES — S780

JUDGED AS DEAD CELL

S781
JUDGED NOT AS DEAD CELL

END

31

# FIG.20

# FIG.21

<table>
<tr><td colspan="2">**INTERNATIONAL SEARCH REPORT**</td><td>International application No.<br>PCT/JP2006/308888</td></tr>
</table>

A.  CLASSIFICATION OF SUBJECT MATTER
*C12M1/34*(2006.01), *C12Q1/04*(2006.01), *G01N21/27*(2006.01), *G01N21/64*
(2006.01), *G01N33/48*(2006.01), *G01N33/483*(2006.01)

According to International Patent Classification (IPC) or to both national classification and IPC

B.  FIELDS SEARCHED

Minimum documentation searched (classification system followed by classification symbols)
*C12M1/34*(2006.01), *C12Q1/04*(2006.01), *G01N21/27*(2006.01), *G01N21/64*
(2006.01), *G01N33/48*(2006.01), *G01N33/483*(2006.01)

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho  1971-2006   Toroku Jitsuyo Shinan Koho   1994-2006

Electronic data base consulted during the international search (name of data base and, where practicable, search terms used)
WPI, JST7580(JDream2), JSTPlus(JDream2)

C.  DOCUMENTS CONSIDERED TO BE RELEVANT

| Category* | Citation of document, with indication, where appropriate, of the relevant passages | Relevant to claim No. |
|---|---|---|
| Y | JP 2005-027623 A  (Olympus Corp.),<br>03 February, 2005 (03.02.05),<br>Claima; Par. Nos. [0038] to [0040], [0044];<br>Figs. 1, 2<br>& US 2004/241832 A1 | 1-22 |
| Y | JP 5-184579 A  (Fujitsu Ltd.),<br>27 July, 1993 (27.07.93),<br>Claims<br>(Family: none) | 1-22 |
| Y | JP 2004-175692 A  (Fuso Pharmaceutical<br>Industries, Ltd.),<br>24 June, 2004 (24.06.04),<br>Par. No. [0018]<br>& US 2005/0075315 A1 | 6,8-12,18,20 |

☒  Further documents are listed in the continuation of Box C.　　☐  See patent family annex.

*　　Special categories of cited documents:
"A"  document defining the general state of the art which is not considered   to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>16 June, 2006 (16.06.06) | Date of mailing of the international search report<br>27 June, 2006 (27.06.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/308888

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2005-532031 A  (Vascular Biogenics Ltd.), 27 October, 2005 (27.10.05), Par. No. [0099] & AU 2400202 A          & CA 2429342 A1 & CN 1592638 A          & EP 1436313 A1 & US 2003/0124100 A1 | 6,8-12,18,20 |
| Y | JP 2002-510291 A  (CASE WESTERN RESERVE UNIVERSITY), 02 April, 2002 (02.04.02), Page 13, lines 6 to 17 & AU 7500398 A          & CA 2290598 A1 & EP 0998294 A1          & WO 98/052577 A1 | 6,8-12,18,20 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H5184579 A
- JP 2000316596 A
- JP 3077628 B
- WO 02052032 A
- JP 2004113175 A

### Non-patent literature cited in the description

- **TOMASI ; MANDUCHI.** Bilateral Filtering for Gray and Color Images. *Proceedings of the 1998 IEEE International Conference on Computer Vision,* 1998
- **VINCENT ; SOILLE.** Watersheds in Digital Spaces: An Efficient Algorithm Based on Immersion Simulations. *IEEE TRANSACTIONS ON PATTERN ANALYSIS AND MACHINE INTELLIGENCE,* June 1991, vol. 13 (6